# EUROPÄISCHE PATENTSCHRIFT

(11) **EP 1 904 497 B1**
(45) Veröffentlichungstag und Bekanntmachung des Hinweises auf die Patenterteilung: **17.08.2011**
(21) Anmeldenummer: 06764095.3
(22) Anmeldetag: 06.07.2006
(51) Int. Cl.: C07D 487/04, H01L 51/50

(54) **SUBSTITUIERTE RYLENDERIVATE**
SUBSTITUTED RYLENE DERIVATIVES
DERIVES DE RYLENE SUBSTITUES

(30) Priorität: 11.07.2005 DE 102005032583
(43) Veröffentlichungstag der Anmeldung: 02.04.2008
(73) Patentinhaber: BASF SE, 67056 Ludwigshafen (DE)
(72) Erfinder: KÖNEMANN, Martin, 68163 Mannheim (DE); BÖHM, Arno, 68305 Mannheim (DE); PSCHIRER, Neil, Gregory, 55116 Mainz (DE); QU, Jianqiang, 67061 Ludwigshafen (DE); MATTERN, Gabriele, 67105 Schifferstadt (DE)
(86) Internationale Anmeldenummer: PCT/EP2006/063955
(87) Internationale Veröffentlichungsnummer: WO 2007/006717

(56) Entgegenhaltungen:
- WO-A-02/14414
- WO-A-97/22607
- WO-A-03/104232

## Beschreibung

Die vorliegende Erfindung betrifft neue Rylenderivate der allgemeinen Formel Ia 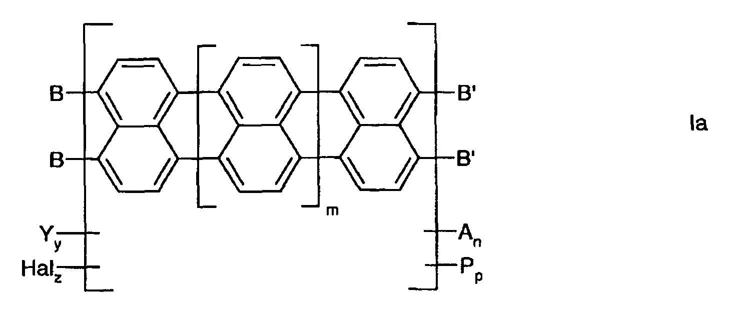 in der die Variablen folgende Bedeutung haben:
- B: miteinander unter Ausbildung eines Sechsrings verbunden zu einem Rest der Formel (a) oder (b) 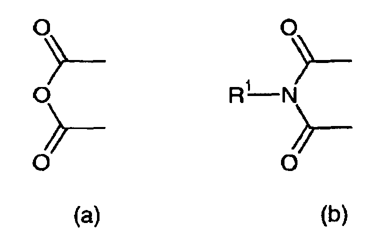 beide Wasserstoff oder ein Rest -COOM oder einer der beiden Reste ein Rest A oder Hal und der andere Rest Wasserstoff;
- B': unabhängig von B miteinander unter Ausbildung eines Sechsrings verbunden zu einem Rest der Formel (a) oder (b) oder beide Wasserstoff oder ein Rest -COOM;
- A: ein Rest der Formel 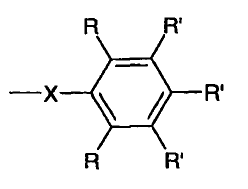 wobei die Reste A für n > 1 gleich oder verschieden sein können;
- Y: ein (Thio)Phenoxyrest der Formel 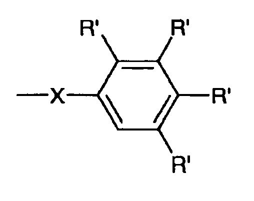 wobei die Reste Y für y > 1 gleich oder verschieden sein können;
- X: -O- oder -S-;
- R: gleiche oder verschiedene Reste: (i) C₁-C₃₀-Alkyl, dessen Kohlenstoffkette durch eine oder mehrere Gruppierungen -O-, -S-, -NR²- und/oder -CO- unterbrochen sein kann und das ein- oder mehrfach durch C₁-C₁₂-Alkoxy, Hydroxy und/oder Halogen substituiert sein kann; wobei höchstens ein Alkylrest R in der 1-Position ein tertiäres Kohlenstoffatom aufweisen kann; (ii) C₃-C₈-Cycloalkyl, das ein- oder mehrfach substituiert sein kann durch C₁-C₁₈-Nkyl und/oder C₁-C₁₂-Alkoxy; wobei höchstens ein Cycloalkylrest R in der 1-position ein tertiäres Kohlenstoffatom aufweisen kann, (iii) Aryl oder Hetaryl, das durch C₁-C₁₈-Alkyl und/oder C₁-C₁₂-Alkoxy ein- oder mehrfach substituiert sein kann; (iv) ein Rest -U-Aryl, der ein- oder mehrfach durch die vorstehenden, als Substituenten für die Arylreste (iii) genannten Reste substituiert sein kann, wobei U eine Gruppierung -O-, -S- oder -NR²- bedeutet; (v) C₁-C₁₂-Alkow, Hydroxy, Halogen oder Cyano
- R': gleiche oder verschiedene Reste: Wasserstoff; einer der für R genannten Reste (i), (ii), (iii), (iv) und (v), wobei die Alkylreste (i) und die Cycloalkylreste (ii) in der 1-Position ein tertiäres Kohlenstoffatom aufweisen können;
- P: ein über ein Stickstoffatom gebundener, 5- bis 9-gliedriger Ring, dessen Kohlenstoffkette durch eine oder mehrere Gruppierungen -O-, -S-, -NR²-, -CO- und/oder -SO₂- unterbrochen sein kann, an den ein oder zwei ungesättigte oder gesättigte 4- bis 8-gliedrige Ringe anneliert sein können, deren Kohlenstoffkette ebenfalls durch diese Gruppierungen und/oder - N= unterbrochen sein kann, wobei das gesamte Ringsystem ein- oder mehrfach substituiert sein kann durch: C₁-C₃₀-Alkyl, das durch Aryl, das durch C₁-C₁₈-Alkyl ein- oder mehrfach substituiert sein kann, ein- oder mehrfach substituiert sein kann;
wobei die Reste P für p > 1 gleich oder verschieden sein können;
- Hal: Fluor, Chlor, Brom oder Iod;
- M: Wasserstoff, Alkalimetallkation, NH₄⁺ oder NR³₄⁺;
- R¹: Wasserstoff; C₁-C₃₀-Alkyl, das ein- oder mehrfach substituiert sein kann durch C₁-C₆- Alkoxy, Cyano und/oder Aryl; Phenyl, das ein- oder mehrfach substituiert sein kann durch C₁-C₁₈-Alkyl, C₁-C₆-Alkoxy, Halogen, Cyano und/oder Nitro;
- R²: Wasserstoff oder C₁-C₁₈-Alkyl, wobei die Reste R² gleich oder verschieden sein können, wenn sie mehrfach auftreten;
- R³: unabhängig voneinander Wasserstoff; C₁-C₁₈-Alkyl, das ein- oder mehrfach durch C₁-C₁₂-Alkoxy, Hydroxy, Halogen und/oder Cyano substituiert sein kann; Aryl oder Hetaryl, das jeweils ein- oder mehrfach durch C1 -C₁₂-Alkyl und/oder die vorstehenden, als Substituenten für Alkyl genannten Reste substituiert sein kann;
- wobei: für m = 0 (Perylenderivate)
- n: 1 bis 4
- p: 0 bis 2, wobei n + p ≤ 4;
- y: 0 bis 4, wobei n + p + y ≤ 4;
- z: 0 bis 4, wobei n + p + y + z ≤ 6;
- und für: m = 1 (Terrylenderivate)
- n: 1 bis 6;
- p: 0 bis 3; wobei n + p ≤ 6;
- y: 0 bis 3, wobei n + p + y ≤ 6;
- z: 0 bis 5, wobei n + p + y + z ≤ 8;
- und für: m = 2 (Quatenylenderivate)
- n: 1 bis 8;
- p: 0 bis 5, wobei n + p ≤ 8;
- y: 0 bis 5, wobei n + p + y ≤ 8;
- z: 0 bis 6, wobei n + p + y ≤ 10.

Außerdem betrifft die Erfindung die Verwendung der Rylenderivate Ia zur Einfärbung von hochmolekularen organischen und anorganischen Materialien, zur Herstellung elektromagnetische Strahlung absorbierender und/oder emittierender wässriger Polymerisatdispersionen, zur Erzeugung für das menschliche Auge nicht sichtbarer, Infrarotlicht absorbierender Markierungen und Beschriftungen, als Infrarotabsorber für das Wärmemanagement, als IR-Laserstrahlenabsorbierende Materialien beim Schweißbehandeln von Kunststoffteilen, zur Lasermarkierung und Laserbeschriftung, als Halbleiter in der organischen Elektronik, als Filter oder Emitter in Display-Anwendungen, als Emitter in Chemilumineszenzanwendungen, als Markierungsgruppen in Nachweisverfahren und als Aktivkomponenten in der Photovoltaik.

Perylentetracarbonsäurediimide, Perylendicarbonsäureimide, die entsprechenden Säureanhydride und die höheren Homologen dieser Verbindungen sind insbesondere als Fluoreszenzfarbmittel und als NIR-Absorber von besonderem Interesse.

Zur Erhöhung ihrer Löslichkeit in den Anwendungsmedien werden üblicherweise Phenoxy- oder Thiophenoxysubstituenten in das Rylengerüst dieser Verbindungen eingeführt. Dabei handelt es sich insbesondere um unsubstituierte oder in p-Stellung z.B. durch tert.-Alkyl substituierte Phenoxy- oder Thiophenoxyreste (WO-A-97/22607, WO-A-031104232, WO-A-96/22332, WO-A-02/76988 sowie die älteren deutschen Patentanmeldungen 10 2005 018 241.0 und 10 2005 021 362.6).

In der WO-A-02/14414 werden außerdem Perylentetracarbonsäuredümide beschrieben, die den Aufbau sternförmiger Polymere ermöglichen. Ihr Perylengerüst ist durch Phenoxyreste substituiert, die wiederum in para-Stellung reaktive Gruppen tragen.

Schließlich werden in der älteren deutschen Patentanmeldung 10 2004 057 585.1 Rylentetracarbonsäurediimide beschrieben, deren Rylengerüst durch cyclische Aminogruppen substituiert ist, die eine bathochrome Verschiebung der Absorption der Diimide bewirken.

Der Erfindung lag die Aufgabe zugrunde, die optischen Eigenschaften von Rylenderivaten weiter zu verbessern, insbesondere auch die Steilheit ihrer Absorptionsbande zu erhöhen.

Demgemäß wurden die Rylenderivate der eingangs definierten Formel Ia gefunden.

Weiterhin wurden bevorzugte Rylenderivate gefunden, bei denen die Variable R in Formel Ia folgende Bedeutung hat:

Besonders bevorzugte Rylenderivate der Formel Ia weisen schließlich folgende Bedeutung der Variablen auf:
- R: gleiche oder verschiedene Reste: (i) C₀-C₃₀-Alkyl, das in der 1-Position kein tertiäres Kohlenstoffatom aufweist, dessen Kohlenstoffkette durch eine oder mehrere Gruppierungen -O-, -S-, -NR²- und/oder -CO- unterbrochen sein kann und das ein- oder mehrfach durch C₁-C₁₂-Alkoxy, Hydroxy und/oder Halogen substituiert sein kann; (ii) C₃-C₈-Cycloalkyl, das in der 1-Position kein tertiäres Kohlenstoffatom aufweist und das ein- oder mehrfach durch C₁-C₁₈-Alkyl und/oder C₁-C₁₂- Alkoxy substituiert sein kann; (iii) Aryl oder Hetaryl, das ein- oder mehrfach durch C₁-C₁₈-Alkyl und/oder C₁- C₁₂-Alkoxy substituiert sein kann; (iv) ein Rest -U-Aryl, der ein- oder mehrfach durch die vorstehenden, als Substituenten für die Arylreste (iii) genannten Reste substituiert sein kann, wobei U eine Gruppierung -O-, -S- oder -NR²- bedeutet; (v) C₁-C₁₂-Alkoxy, Hydroxy, Halogen oder Cyano;
- R': gleiche oder verschiedene Reste: Wasserstoff; einer der für R genannten Reste (i), (ii), (iii), (iv) und (v), wobei die Alkylreste (i) und die Cycloalkylreste (ii) in der 1-Position ein tertiäres Kohlenstoffatom aufweisen können;
- R²: Wasserstoff oder C₁-C₁₈-Alkyl;
- R³: unabhängig voneinander Wasserstoff; C₁-C₁₈-Alkyl, das ein- oder mehrfach durch C₁-C₁₂-Alkoxy, Hydroxy, Halogen und/oder Cyano substituiert sein kann; Aryl oder Hetaryl, das jeweils ein- oder mehrfach durch C₁-C₁₂-Alkyl und/oder die vorstehenden, als Substituenten für Alkyl genannten Reste substituiert sein kann;

Besonders bevorzugte Rylencerivate der Formel Ia weisen schließlich folgende Bedeutung der Variablen auf:
- A: ein Rest der Formel 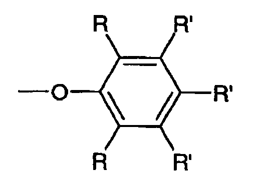
wobei die Reste A für n > 1 gleich sind;
- R: gleiche oder verschiedene Reste:
(i) C₁-C₁₈-Alkyl, das in der 1-Position kein tertiäres Kohlenstoffatom aufweist, dessen Kohlenstoffkette durch eine oder mehrere Gruppierungen -O-, -S-, -NR²- und/oder -CO- unterbrochen sein kann und das ein- oder mehrfach durch C₁-C₁₂-Alkoxy substituiert sein kann;
(ii) C₃-C₈-Cycloalkyl, das in der 1-Position kein tertiäres Kohlenstoffatom aufweist und ein- oder mehrfach durch C₁-C₁₈-Alkyl und/oder C₁-C₁₂-Alkoxy substituiert sein kann;
(iii) Aryl oder Hetaryl, das jeweils ein- oder mehrfach durch C₁-C₁₈-Alkyl, C₁- C₁₂-Alkoxy, Hydroxy und/oder Halogen substituiert sein kann;
(iv) ein Rest -U-Aryl, der ein- oder mehrfach durch die vorstehenden, als Substituenten für die Arylreste (iii) genannten Reste substituiert sein kann, wobei U eine Gruppierung -O-, -S- oder -NR²- bedeutet;
(v) C₁-C₁₂-Alkoxy, Hydroxy, Halogen oder Cyano;
- R': gleiche oder verschiedene Reste: Wasserstoff; einer der für R genannten Reste (i), (ii), (iii), (iv) und (v), wobei die Alkylreste (i) und die Cycloalkylreste (ii) in der 1-Position ein tertiäres Kohlenstoffatom enthalten können;
- R²: Wasserstoff oder C₁-C₆-Alkyl;
- R³: unabhängig voneinander Wasserstoff; C₁-C₁₈-Alkyl, das ein- oder mehrfach durch C₁-C₆-Alkoxy, Hydroxy, Halogen und/oder Cyano substituiert sein kann; Aryl oder Hetaryl, das jeweils ein- oder mehrfach durch C₁-C₁₂-Alkyl und/oder die vorstehenden, als Substituenten für Alkyl genannten Reste substituiert sein kann;
- n: 1 bis 6.

Bevorzugte Reste R¹ sind ortho,ortha'-dialkylsubstituierte Arylreste, vor allem solche, die auch Bestandteil des Restes A sind, oder lineare Alkylketten, die über ein mittleres Kohlenstoffatom an das Imidstickstoffatom gebunden sind. Ausgewählte Beispiele für diese Reste R¹ sind 2,6-Dimethylphenyl, 2,6-Diisopropylphenyl, 2,6-Diisopropyl-4-butylphenyl, 5-Undecyl, 7-Tridecyl und 9-Pentadecyl.

M bedeutet schließlich Wasserstoff, ein Alkalimetallkation, NH₄⁺ oder NR³₄⁺, wobei Alkalimetallkationen, wie Lithium-, Natrium-, Kalium- oder Caesiumkationen, besonders bevorzugt sind.

Als Beispiele für bevorzugte Rylenderivate I seien Perylen-, Terrylen- und Quaterrylenderivate genannt.

Beispiele für die bevorzugten funktionalisierten Rylenderivate sind:

Perylen-3,4:9,10-tetracarbonsäurediimide, Perylentetracarbonsäuren, deren Salze und Anhydride, Perylentetracarbonsäuremonoimidmonoanhydride und Kondensationsprodukte von Perylentetracarbonsäure(dianhydride)n oder Perylentetracarbonsäuremonoimidmonoanhydriden mit aromatischen α,ω-Diaminen, insbesondere mit 1,2-Diaminobenzol- und 1,8-Diaminonaphthalinderivaten;

Terrylen-3,4:11,12-tetracarbonsäurediimide, Terrylentetracarbonsäuren, deren Salze und Anhydride, Terrylentetracarbonsäuremonoimidmonoanhydride und Kondensationsprodukte von Tenylentetracarbonsäure(dianhydride)n oder Terrylentetracarbonsäuremonoimidmonoanhydriden mit aromatischen α,ω-Diaminen, insbesondere mit 1,2-Diaminobenzol- und 1,8-Diaminonaphthalinderivaten;

Quaterrylen-3,4:13,14-tetracarbonsäurediimide, Quaterrylentetracarbonsäuren, deren Salze und Anhydride, Quatenylentetracarbonsäuremonoimidmonoanhydride und Kondensationsprodukte von Quaterrylentetracarbonsäure(dianhydride)n oder Quaterrylentetracarbonsäuremonoimidmonoanhydriden mit aromatischen α,ω-Diaminen, insbesondere mit 1,2-Diaminobenzol- und 1,8-Diaminonaphthalinderivaten;

Perylen-3,4-dicarbonsäureimide und Perylendicarbonsäuren, deren Salze, Anhydride und Kondensationsprodukte mit aromatischen α,ω-Diaminen, insbesondere mit 1,2-Diaminobenzol- und 1,8-Diaminonaphthalinderivaten;

Terrylen-3,4-dicarbonsäureimide und Terrylendicarbonsäuren, deren Salze, Anhydride und Kondensationsprodukte mit aromatischen α,(-Diaminen, insbesondere mit 1,2-Diaminobenzol- und 1,8-Diaminonaphthalinderivaten;

Quaterrylen-3,4-dicarbonsäureimide und Quaterrylendicarbonsäuren, deren Salze, Anhydride und Kondensationsprodukte mit aromatischen (,(-Diaminen, insbesondere mit 1,2-Diaminobenzol- und 1,8-Diaminonaphthalinderivaten.

Bei den Kondensationsprodukten der Rylentetracarbonsäuren bzw. der Rylentetracarbonsäuredianhydride kann es sich um Mono- oder Dikondensationsprodukte handeln. Die Dikondensationsprodukte können in symmetrischer oder asymmetrischer Form vorliegen, in der Regel liegen Gemische beider Formen vor.

Besonders bevorzugt sind die genannten Rylentetracarbonsäurediimide und Rylendicarbonsäureimide, wobei die Rylentetracarbonsäurediimide ganz besonders bevorzugt sind.

Die erfindungsgemäßen Rylenderivate Ia sind durch mindestens einen Rest A der Formel 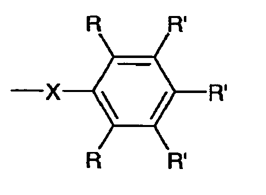 substituiert.

Die (Thio)Phenoxyreste A sind in beiden ortho-Positionen durch Reste R substituiert. Die beiden Reste R können gleich oder verschieden sein, bevorzugt sind sie jedoch gleich.

Die (Thio)Phenoxyreste A können auch in einer, zweien oder allen drei weiteren Ringpositionen durch gleiche oder ungleiche, von Wasserstoff verschiedene Reste R' substituiert sein. Bevorzugt ist dabei eine zusätzliche Substitution in der para-Position.

Bei den Resten R kann es sich, wie eingangs ausgeführt, um Alkylreste (i) und Cycloalkylreste (ii), wobei höchstens einer der Reste in der 1-Position ein tertiäres Kohlenstoffatom aufweisen darf, vorzugsweise jedoch keiner der Reste in der
1.Position ein tertiäres Kohlenstoffatom aufweist, Aryl- oder Hetarylreste (iii), die über eine Gruppierung -O-, -S- oder -NR²- an den (Thio)Phenoxyrest gebunden sein können (iv), oder um die Substituenten (v) handeln.

Mögliche Reste R' entsprechen den Resten R, wobei Alkylreste und Cycloalkylreste hier auch ohne Einschränkung tertiäre Kohlenstoffatome in der 1-Position aufweisen können.

Bevorzugte Reste R' sind dabei die Alkyl-, Cycloalkyl- und Phenylreste, vor allem die Alkylreste R' mit sekundärem oder primärem Kohlenstoffatom in der 1-Position sowie Methyl und die Cycloalkylreste R' mit sekundärem Kohlenstoffatom in der 1-Position, wobei die Alkyl- und Cycloalkylreste mit sekundärem Kohlenstoffatom in der 1-Position besonders hervorzuheben sind.

Beispiele für die in den erfindungsgemäßen Formeln auftretenden Reste R und R' sowie R² bis R³ sind weiter unten aufgelistet.

Beispiele für ganz besonders bevorzugte Reste A sind:
2,6-Dimethylphenoxy, 2,6-Diethylphenoxy, 2,6-Diisopropylphenoxy, 2,6-Di(2-butyl)-phenoxy, 2,6-Di(n-butyl)phenoxy, 2,6-Di(2-hexyl)phenoxy, 2,6-Di(n-hexyl)phenoxy, 2,6-Di(2-dodecyl)phenoxy, 2,6-Di(n-dodecyl)phenoxy, 2,6-Dicyclohexylphenoxy, 2,6-Diphenylphenoxy, 2,6-Dimethyl-4-(n-butyl)phenoxy, 2,6-Diethyl-4-(n-butyl)phenoxy, 2,6-Diisopropyl-4-(n-butyl)phenoxy, 2,6-Di(2-butyl)-4-(n-butyl)phenoxy, 2,4,6-Tri(n-butyl)phenoxy, 2,6-Di(2-hexyl)-4-(n-butyl)phenoxy, 2,6-Di(n-hexyl)-4-(n-butyl)phenoxy, 2,6-Di(2-dodecyl)-4-(n-butyl)phenoxy, 2,6-Di(n-dodecyl)-4-(n-butyl)phenoxy, 2,6-Dicyclohexy!-4-(n-butyl)phenoxy, 2,6-Diphenyl-4-(n-butyl)phenoxy, 2,6-Dimethyl-4-(n-nonyl)phenoxy, 2,6-Diethyl-4-(n-nonyl)phenoxy, 2,6-Diisopropyl-4-(n-nonyl)phenoxy, 2,6-Di(2-butyl)-4-(n-nonyl)phenoxy, 2,6-Di(2-butyl)-4-(n-nonyl)phenoxy, 2,6-Di(2-hexyl)-4-(n-nonyl)phenoxy, 2,6-Di(n-hexyl)-4-(n-nonyl)phenoxy, 2,6-Di(2-dodecyl)-4-(n-nonyl)-phenoxy, 2,6-Di(n-dodecyl)-4-(n-nonyl)phenoxy, 2,6-Dicyclohexyl-4-(n-nonyl)phenoxy, 2,6-Diphenyl-4-(n-nonyl)phenoxy, 2,6-Dimethyl-4-(n-octadecyl)phenoxy, 2,6-Diethyl-4-(n-octadecyl)phenoxy, 2,6-Diisopropyl-4-(n-octadecyl)phenoxy, 2,6-Di(2-butyl)-4-(n-octadecyl)phenoxy, 2,6-Di(2-butyl)-4-(n-octadecyl)phenoxy, 2,6-Di(2-hexyl)-4-(n-octadecyl)phenoxy, 2,6-Di(n-hexyl)-4-(n-octadecyl)phenoxy, 2,6-Di(2-dodecyl)-4-(n-octadecyl)phenoxy, 2,6-Di(n-dodecyl)-4-(n-octadecyl)phenoxy, 2,6-Dicyclohexyl-4-(n-octadecyl)phenoxy,2,6-Dimethyl-4-(tert.-butyl)phenoxy, 2,6-Diethyl-4-(tert.-butyl)phenoxy, 2,6-Diisopropyl-4-(tert.-butyl)phenoxy, 2,6-Di(2-butyl)-4-(tert.-butyl)phenoxy, 2,6-Di-(n-butyl)-4-(tert.-butyl)phenoxy, 2,6-Di(2-hexyl)-4-(tert.-butyl)phenoxy, 2,6-Di(n-hexyl)-4-(tert.-butyl)phenoxy, 2,6-Di(2-dodecyl)-4-(tert.-butyl)phenoxy, 2,6-Di(n-dodecyl)-4-(tert.-butyl)phenoxy, 2,6-Dicyclohexyl-4-(tert.-butyl)phenoxy, 2,6-Diphenyl-4-(tert.-butyl)-phenoxy, 2,6-Dimethyl-4-(tert.-octyl)phenoxy, 2,6-Diethyl-4-(tert.-octyl)phenoxy, 2,6-Diisopropyl-4-(tert.-octyl)phenoxy, 2,6-Di(2-butyl)-4-(tert.-octyl)phenoxy, 2,6-Di-(n-butyl)-4-(tert.-octyl)phenoxy, 2,6-Di(2-hexyl)-4-(tert.-octyl)phenoxy, 2,6-Di(n-hexyl)-4-(tert.-octyl)phenoxy, 2,6-Di(2-dodecyl)-4-(tert.-octyl)phenoxy, 2,6-Di(n-dodecyl)-4-(tert.-octyl)phenoxy, 2,6-Dicyclohexyl-4-(tert.-octyl)phenoxy und 2,6-Diphenyl-4-(tert.-octyl)-phenoxy;
2,6-Dimethylthiophenoxy, 2,6-Diethylthiophenoxy, 2,6-Diisopropylthiophenoxy, 2,6-Di(2-butyl)thiophenoxy, 2,6-Di(n-butyl)thiophenoxy, 2,6-Di(2-hexyl)thiophenoxy, 2,6-Di(n-hexyl)thiophenoxy, 2,6-Di(2-dodecyl)thiophenoxy, 2,6-Di(n-dodecyl)thiophenoxy, 2,6-Dicyclohexylthiophenoxy, 2,6-Diphenylthiophenoxy, 2,6-Dimethyl-4-(n-butyl)thiophenoxy, 2,6-Diethyl-4-(n-butyl)thiophenoxy, 2,6-Diisopropyl-4-(n-butyl)thiophenoxy, 2,6-Di(2-butyl)-4-(n-butyl)thiophenoxy, 2,4,6-Tri(n-butyl)thiophenoxy, 2,6-Di(2-hexyl)-4-(n-butyl)thiophenoxy, 2,6-Di(n-hexyl)-4-(n-butyl)thiophenoxy, 2,6-Di(2-dodecyl)-4-(n-butyl)thiophenoxy, 2,6-Di(n-dodecyl)-4-(n-butyl)thiophenoxy, 2,6-Dicyclohexyl-4-(n-butyl)thiophenoxy, 2,6-Diphenyl-4-(n-butyl)thiophenoxy, 2,6-Di-methyl-4-(n-nonyl)thiophenoxy, 2,6-Diethyl-4-(n-nonyl)thiophenoxy, 2,6-Diisopropyl-4-(n-nonyl)thiophenoxy, 2,6-Di(2-butyl)-4-(n-nonyl)thiophenoxy, 2,6-Di(2-butyl)-4-(n-nonyl)thiophenoxy 2,6-Di(2-hexyl)-4-(n-nonyl)thiophenoxy, 2,6-Di(n-hexyl)-4-(n-nonyl)thiophenoxy, 2,6-Di(2-dodecyl)-4-(n-nonyl)thiophenoxy, 2,6-Di(n-dodecyl)-4-(n-nonyl)thiophenoxy, 2,6-Dicyclohexyl-4-(n-nonyl)thiophenoxy, 2,6-Diphenyl-4-(n-nonyl)thiophenoxy, 2,6-(Dimethyl)-4-(n-octadecyl)thiophenoxy, 2,6-(Diethyl)-4-(n-octadecyl)thiophenoxy, 2,6-Diisopropyl-4-(n-octadecyl)thiophenoxy, 2,6-Di(2-butyl)-4-(n-octadecyl)thiophenoxy, 2,6-Di(2-butyl)-4-(n-octadecyl)thiophenoxy, 2,6-Di(2-hexyl)-4-(n-octadecyl)thiophenoxy, 2,6-Di(n-hexyl)-4-(n-octa-decyl)thiophenoxy, 2,6-Di(2-dodecyl)-4-(n-octadecyl)thiophenoxy, 2,6-Di(n-dodecyl)-4-(n-octadecyl)thiophenoxy, 2,6-Dicyclohexyl-4-(n-octadecyl)thiophenoxy, 2,6-Dimethyl-4-(tert.-butyl)thiophenoxy, 2,6-Diethyl-4-(tert.-butyl)-thiophenoxy, 2,6-Diisopropyl-4-(tert.-butyl)thiophenoxy, 2,6-Di(2-butyl)-4-(tert.-butyl)-thiophenoxy, 2,6-D1-(n-butyl)-4-(tert.-butyl)thiophenoxy, 2,6-Di(2-hexyl)-4-(tert.-butyl)-thiophenoxy, 2,6-Di(n-hexyl)-4-(tert.-butyl)thiophenoxy, 2,6-Di(2-dodecyl)-4-(tert.-butyl)-thiophenoxy, 2,6-Di(n-dodecyl)-4-(tert.-butyl)thiophenoxy, 2,6-Dicyclohexyl-4-(tert.-butyl)thiophenoxy, 2,6-Diphenyl-4-(tert.-butyl)thiophenoxy, 2,6-Dimethyl-4-(tert.-octyl)-thiophenoxy, 2,6-Diethyl-4-(tert.-octyl)thiophenoxy, 2,6-Diisopropyl-4-(tert.-octyl)thiophenoxy, 2,6-Di(2-butyl)-4-(tert.-octyl)thiophenoxy, 2,6-Di-(n-butyl)-4-(tert.-octyl)thiophenoxy, 2,6-Di(2-hexyl)-4-(tert.-octyl)thiophenoxy, 2,6-Di(n-hexyl)-4-(tert.-octyl)thiophenoxy, 2,6-Di(2-dodecyl)-4-(tert.-octyl)thiophenoxy, 2,6-Di(n-dodecyl)-4-(tert.-octyl)-thiophenoxy, 2,6-Dicyclohexyl-4-(tert.-octyl)thiophenoxy und 2,6-Diphenyl-4-(tert.-octyl)thiophenoxy.

Neben den erfindungsgemäßen Resten A können die Rylenderivate Ia weitere Substituenten im Rylengerüst tragen.

Weitere mögliche Substituenten sind z.B. (Thio)Phenoxyreste Y 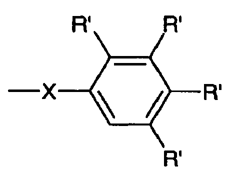 die in mindestens einer ortho-Position nicht substituiert sind und vorzugsweise überhaupt keine Substituenten tragen (R' = H).

Weitere mögliche Substituenten sind Halogenatome, wie Fluor, Chlor, Brom oder Iod, vor allem Chlor oder Brom. In der Regel sind Halogenatome dann in den Rylenderivaten Ia enthalten, wenn der Austausch der zuerst in das Rylengerüst eingeführten Halogenatome durch die Reste A (oder Y) nicht vollständig erfolgt ist.

Schließlich können die Rylenderivate Ia auch durch cyclische Aminogruppen P substituiert sein.

Bei dem Substituenten P handelt es sich um einen über ein Stickstoffatom gebundenen, 5- bis 9-gliedrigen Ring, dessen Kohlenstoffkette durch eine oder mehrere Gruppierungen -O-, -S-, -NR²-, -CO- und/oder -SO₂- unterbrochen sein kann, an den ein oder zwei ungesättigte oder gesättigte 4- bis 8-gliedrige Ringe anneliert sein können, deren Kohlenstoffkette ebenfalls durch diese Gruppierungen und/oder- N= unterbrochen sein kann, wobei das gesamte Ringsystem ein- oder mehrfach substituiert sein kann durch:
C₁-C₃₀-Alkyl, das durch Aryl, das ein- oder mehrfach durch C₁-C₁₈-Alkyl substituiert sein kann, ein- oder mehrfach substituiert sein kann;

Bevorzugt bedeutet P einen über ein Stickstoffatom gebundenen, 5- bis 7-gliedrigen Ring, dessen Kohlenstoffkette durch eine oder mehrere Gruppierungen -O-, -S-, -NR²-, -CO- und/oder -SO₂- unterbrochen sein kann, an den ein oder zwei ungesättigte oder gesättigte 4- bis 8-gliedrige Ringe anneliert sein können, deren Kohlenstoffkette ebenfalls durch diese Gruppierungen und/oder- N= unterbrochen sein kann, wobei das gesamte Ringsystem ein- oder mehrfach substituiert sein kann durch C₁-C₂₄-Alkyl, das durch Aryl, das C₁-C₁₈-Alkyl als Substituenten tragen kann, substituiert sein kann.

Besonders bevorzugte Reste P basieren auf den cyclischen Aminen Piperidin, Pyrrolidin, Piperazin, Morpholin und Thiomorpholin (1,4-Thiazin), wobei die Piperidine, Pyrrolidine, Piperazine und Morpholine bevorzugt und die Piperidine besonders bevorzugt sind.

Die cyclischen Basisamine können chemisch modifiziert sein, vorzugsweise sind sie jedoch nicht modifiziert. Als Beispiele für chemisch modifizierte Basisamine seien im einzelnen genannt:
Piperidin, 2- und 3-Methylpiperidin, 6-Ethylpiperidin, 2,6- und 3,5-Dimethylpiperidin, 2,2,6,6-Tetramethylpiperidin, 4-Benzylpiperidin, 4-Phenylpiperidin, Piperidin-4-ol, Piperidin-4-carbonsäure, Piperidin-4-carbonsäuremethylester, Piperidin-4-carbonsäure-ethylester, Piperidin-4-carbonsäureamid, 2,2,6,6-Tetramethylpiperidin-4-on, 2,2,6,6-Tetramethylpiperidin-4-ylamin, Decahydrochinolin und Decahydroisochinolin;
Pyrrolidin, 2-Methylpyrrolidin, 2,5-Dimethylpyrrolidin, 2,5-Diethylpyrrolidin, Tropanol, Pyrrolidin-2-carbonsäuremethylester, Pyrrolidin-2-carbonsäureethylester, Pyrrolidin-2-carbonsäurebenzylester, Pyrrolidin-2-carbonsäureamid, 2,2,5,5-Tetramethylpyrrolidin-3-carbonsäure, 2,2,5,5-Tetramethylpyrrolidin-3-carbonsäuremethylester, 2,2,5,5-Tetramethylpyrrolidin-3-carbonsäureethylester, 2,2,5,5-Tetramethylpyrrolidin-3-carbonsäurebenzylester, Pyrrolidin-3-ylamin, (2,6-Dimethylphenyl)pyrrolidin-2-ylmethylamin, (2,6-Diisopropylphenyl)pyrrolidin-2-ylmethylamin und Dodecahydrocarbazol;
Piperazin, Diketopiperazin; 1-Benzylpiperazin, 1-Phenethylpiperazin, 1-Cyclohexylpiperazin, 1-Phenylpiperazin, 1-(2,4-Dimethylphenyl)piperazin, 1-(2-,3-und4-Methoxyphenyl)piperazin, 1-(2-, 3- und 4-Ethoxyphenyl)piperazin, 1-(2-, 3- und 4-Fluorphenyl)piperazin, 1-(2-, 3- und 4-Chlorphenyl)piperazin, 1-(2-, 3- und 4-Bromphenyl)-piperazin, 1-, 2- und 3-Pyridin-2-ylpiperazin und 1-Benzo[1,3]dioxol-4-ylmethylpiperazin;
Morpholin, 2,6-Dimethylmorpholin, 3,3,5,5-Tetramethylmorpholin, Morpholin-2- und -3-ylmethanol, Morpholin-2- und -3-ylessigsäure, Morpholin-2-und -3-ylessigsäuremethylester, Morpholin-2- und -3-ylessigsäureethylester, 3-Morpholin-3-ylpropionsäuremethylester, 3-Morpholin-3-ylpropionsäureethylester, 3-Morpholin-3-ylpropionsäure-tert.-butylester, Morpholin-2- und -3-ylacetamid, 3-Morpholin-3-yl-propionsäure-amid, 3-Benzylmorpholin, 3-Methyl-2-phenylmorpholin, 2- und 3-Phenylmorpholin, 2-(4-Methoxyphenyl)morpholin, 2-(4-Trifluoromethylphenyl)morpholin, 2-(4-Chlorphenyl)-morpholin, 2-(3,5-Dichlorphenyl)morpholin, Morpholin-2- und -3-carbonsäure, Morpholin-3-carbonsäuremethylester, 3-Pyridin-3-ylmorpholin, 5-Phenylmorpholin-2-on, 2-Mor-pholin-2-ylethylamin und Phenoxazin;
Thiomorpholin, 2- und 3-Phenylthiomorpholin, 2- und 3-(4-Methoxyphenyl)thiomorpholin, 2- und 3-(4-Fluorphenyl)thiomorpholin, 2- und 3-(4-Trifluormethylphenyl)thiomorpholin, 2- und 3-(2-Chlorphenyl)thiomorpholin, 4-(2-Aminoethyl)thiomorpholin, 3-Pyridin-3-ylthiomorpholin, 3-Thiomorpholincarbonsäure, 6,6-Dimethyl-5-oxo-3-thiomorpholincarbonsäure, 3-Thiomorpholinon und 2-Phenylthiomorpholin-3-on sowie die Thiomorpholinoxide und -dioxide.

Durch Einführung der Substituenten P kann eine zusätzliche bathochrome Verschiebung der Absorption der Rylenderivate Ia bewirkt werden.

Die Substitution der Rylenderivate Ia durch die oben aufgeführten Reste soll am Beispiel der Perylen-, Terrylen-, und Quaterrylenderivate näher erläutert werden.

So entsprechen die erfindungsgemäßen Perylen-, Terrylen- und Quaterrylenderivate der Formel la 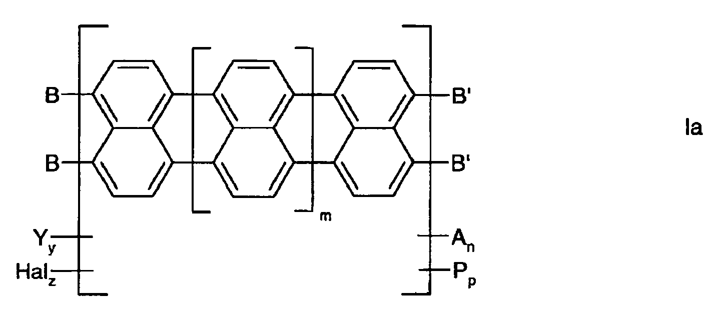 mit:
- B: miteinander unter Ausbildung eines Sechsrings verbunden zu einem Rest der Formel (a) oder (b) 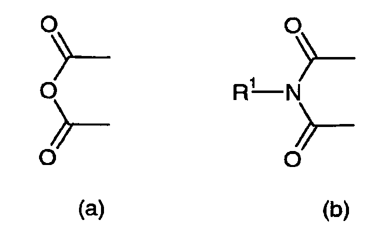 beide Wasserstoff oder ein Rest -COOM oder einer der beiden Reste ein Rest A oder Hal und der andere Rest Wasserstoff;
- B': unabhängig von B miteinander unter Ausbildung eines Sechsrings verbunden zu einem Rest der Formel (a) oder (b) oder beide Wasserstoff oder ein Rest - COOM;

Die Variablen n (Anzahl der Substituenten A), p (Anzahl der cyclischen Aminoreste P), y (Anzahl der (Thio)Phenoxyreste Y) und z (Anzahl der Halogenatome Hal) haben dabei für die einzelnen Rylenderivate Ia folgende Bedeutung:
Perylenderivate Ia (m = 0):
   - n: 1 bis 4;
   - p: 0 bis 2, wobei n + p ≤ 4;
   - y: 0 bis 4, wobei n + p + y ≤ 4;
   - z: 0 bis 4, wobei n + p + y + z ≤ 6.
Tenylenderivate Ia (m = 1):
   - n: 1 bis 6;
   - p: 0 bis 3, wobei n + p ≤ 6;
   - y: 0 bis 3, wobei n + p + y ≤ 6;
   - z: 0 bis 5, wobei n + p + y + z ≤ 8.
Quaterrylenderivate la (m = 2):
   - n: 1 bis 8;
   - p: 0 bis 5, wobei n + p ≤ 8;
   - y: 0 bis 5, wobei n + p + y ≤ 8;
   - z: 0 bis 6, wobei n + p + y + z ≤ 10.
Besonders bevorzugt sind folgende Bedeutungen der Variablen n, p, y und z:
Perylenderivate Ia (m = 0):
   - n: 1 oder 2;
   - p: 0;
   - y: 0;
   - z: 0.
Terrylenderivate Ia (m = 1):
   - n: 1 bis 4;
   - p: 0;
   - y: 0;
   - z: 0 bis 3, wobei gilt: n + z ≤ 4.
Quatenytendenvate Ia (m = 2):
   - n: 1 bis 6;
   - p: 0;

   - y: 0;
   - z: 0 bis 5, wobei gilt: n +z ≤ 6.

Bevorzugt sind die Perylentetracarbonsäurederivate Ia zweifach (vor allem in 1,7- oder 1,6-Position) oder vierfach (vor allem in 1,6,7,12-Position) substituiert und enthalten vorzugsweise 2 Reste A. Bei den Perylendicarbonsäurederivaten Ia ist eine zusätzliche Substitution der peri-Position (9-Position) insbesondere durch einen Rest A oder ein Halogenatom möglich.

Die Terrylentetracarbonsäurederivate la sind vorzugsweise vierfach (vor allem in 1,6,9,14-Position) substituiert und enthalten insbesondere mindestens 2 Reste A. Auch bei den Terrylendicarbonsäurederivaten ist eine peri-Substitution (11-Position) vor allem durch ein Halogenatom möglich.

Schließlich sind die Quaterrylentetracarbonsäurederivate la bevorzugt vierfach (vor allem in 1,6,11,16-Position) oder sechsfach (vor allem in 1,6,8,11,16,18- oder 1,6,8,11,16,19-Position) substituiert und enthalten vorzugsweise 4 Reste A. Peri-Substitution (13-Position) insbesondere durch ein Halogenatom ist bei den Quaterrylendicarbonsäurederivaten wiederum zusätzlich möglich.

Häufig fallen die Rylenderivate Ia in Form von Mischungen von Produkten mit unterschiedlichem Substitutionsgrad an, in denen die oben explizit genannten Produkte jeweils den Hauptanteil ausmachen.

Besonders bevorzugte Rylenderivate Ia sind die Perylen-, Terrylen- und Quaterrylentetracarbonsäurediimide und die Perylen-, Terrylen- und Quaterrylendicarbonsäure-imide, wobei die Rylentetracarbonsäurediimide ganz besonders bevorzugt sind.

Insbesondere bevorzugt sind Diimide und Monoimide Ia, die an den Imidstickstoffatomen durch Arylreste substituiert sind, die ebenfalls eine ortho,ortho'-Disubstitution aufweisen, also insbesondere den in den Resten A enthaltenen Phenylresten entsprechen, da diese Rylenderivate la besonders steile Absorptionsbanden zeigen.

Schließlich seien als Beispiele für die in den erfindungsgemäßen Formeln auftretenden Reste R, R' und R¹ sowie deren Substituenten im Einzelnen genannt:
Methyl, Ethyl, Propyl, Isopropyl, Butyl, Isobutyl, tert.-Butyl, Pentyl, Isopentyl, Neopentyl, tert.-Pentyl, Hexyl, 2-Methylpentyl, Heptyl, 1-Ethylpentyl, Octyl, 2-Ethylhexyl, Isooctyl, Nonyl, Isononyl, Decyl, Isodecyl, Undecyl, Dodecyl, Tridecyl, Isotridecyl, Tetradecyl, Pentadecyl, Hexadecyl, Heptadecyl, Octadecyl, Nonadecyl und Eicosyl (die obigen Bezeichnungen Isooctyl, Isononyl, Isodecyl und Isotridecyl sind Trivialbezeichnungen und stammen von den nach der Oxosynthese erhaltenen Alkoholen);
2-Methoxyethyl, 2-Ethoxyethyl, 2-Propoxyethyl, 2-Isopropoxyethyl, 2-Butoxyethyl, 2- und 3-Methoxypropyl, 2- und 3-Ethoxypropyl, 2- und 3-Propoxypropyl, 2- und 3-Butoxypropyl, 2- und 4-Methoxybutyl, 2- und 4-Ethoxybutyl, 2- und 4-Propoxybutyl, 3,6-Dioxaheptyl, 3,6-Dioxaoctyl, 4,8-Dioxanonyl, 3,7-Dioxaoctyl, 3,7-Dioxanonyl, 4,7-Dioxaoctyl, 4,7-Dioxanonyl, 2- und 4-Butoxybutyl, 4,8-Dioxadecyl, 3,6,9-Trioxadecyl, 3,6,9-Trioxaundecyl, 3,6,9-Trioxadodecyl, 3,6,9,12-Tetraoxatridecyl und 3,6,9,12-Tetraoxatetradecyl;
2-Methylthioethyl, 2-Ethylthioethyl, 2-Propylthioethyl, 2-Isopropylthioethyl, 2-Butylthioethyl, 2- und 3-Methylthiopropyl, 2- und 3-Ethylthiopropyl, 2- und 3-Propylthiopropyl, 2- und 3-Butylthiopropyl, 2- und 4-Methylthiobutyl, 2- und 4-Ethylthiobutyl, 2- und 4-Propylthiobutyl, 3,6-Dithiaheptyl, 3,6-Dithiaoctyl, 4,8-Dithianonyl, 3,7-Dithiaoctyl, 3,7-Dithianonyl, 2- und 4-Butylthiobutyl, 4,8-Dithiadecyl, 3,6,9-Trithiadecyl, 3,6,9-Trithiaundecyl, 3,6,9-Trithiadodecyl, 3,6,9,12-Tetrathiatridecyl und 3,6,9,12-Tetrathiatetradecyl;
2-Monomethyl- und 2-Monoethylaminoethyl, 2-Dimethylaminoethyl, 2- und 3- Dimethylaminopropyl, 3-Monoisopropylaminopropyl, 2- und 4-Monopropylaminobutyl, 2- und 4-Dimethylaminobutyl, 6-Methyl-3,6-diazaheptyl, 3,6-Dimethyl-3,6-diazaheptyl, 3,6-Diazaoctyl, 3,6-Dimethyl-3,6-diazaoctyl, 9-Methyl-3,6,9-triazadecyl, 3,6,9-Trimethyl-3,6,9-triazadecyl, 3,6,9-Triazaundecyl, 3,6,9-Trimethyl-3,6,9-triazaundecyl, 12-Methyl-3,6,9,12-tetraazatridecyl und 3,6,9,12-Tetramethyl-3,6,9,12-tetraazatridecyl;
(1-Ethylethyliden)aminoethylen, (1-Ethylethyliden)aminopropylen, (1-Ethylethyliden)-aminobutylen, (1-Ethylethyliden)aminodecylen und (1-Ethylethyliden)aminododecylen;
Propan-2-on-1-yl, Butan-3-on-1-yl, Butan-3-on-2-yl und 2-Ethylpentan-3-on-1-yl;
2-Methylsulfoxidoethyl, 2-Ethylsulfoxidoethyl, 2-Propylsulfoxidoethyl, 2-Isopropylsulf oxidoethyl, 2-Butylsulfoxidoethyl, 2- und 3-Methylsulfoxidopropyl, 2- und 3-Ethylsulf oxidopropyl, 2- und 3-Propylsulfoxidopropyl, 2- und 3-Butylsulfoxidopropyl, 2- und 4-Methylsulfoxidobutyl, 2- und 4-Ethylsulfoxidobutyl, 2- und 4-Propylsulfoxidobutyl und 4-Butylsulfoxidobutyl;
2-Methylsulfonylethyl, 2-Ethylsulfonylethyl, 2-Propylsulfonylethyl, 2-Isopropylsulfonylethyl, 2-Butylsulfonylethyl, 2- und 3-Methylsulfonylpropyl, 2- und 3-Ethylsulfonylpropyl, 2- und 3-Propylsulfonylpropyl, 2- und 3-Butylsulfonylproypl, 2- und 4-Methylsulfonylbutyl, 2- und 4-Ethylsulfonylbutyl, 2- und 4-Propylsulfonylbutyl und 4-Butylsulfonylbutyl;
Carboxymethyl, 2-Carboxyethyl, 3-Carboxypropyl, 4-Carboxybutyl, 5-Carboxypentyl, 6-Carboxyhexyl, 8-Carboxyoctyl, 10-Carboxydecyl, 12-Carboxydodecyl und 14-Carboxytetradecyl;
Sulfomethyl, 2-Sulfoethyl, 3-Sulfopropyl, 4-Sulfobutyl, 5-Sulfopentyl, 6-Sulfohexyl, 8-Sulfooctyl, 10-Sulfodecyl, 12-Sulfododecyl und 14-Sulfotetradecyl;
2-Hydroxyethyl, 2- und 3-Hydroxypropyl, 1-Hydroxyprop-2-yl, 3- und 4-Hydroxybutyl, 1-Hydroxybut-2-yl und 8-Hydroxy-4-oxaoctyl;
2-Cyanoethyl, 3-Cyanopropyl, 3- und 4-Cyanobutyl, 2-Methyl-3-ethyl-3-cyanopropyl, 7-Cyano-7-ethylheptyl und 4-Methyl-7-methyl-7-cyanoheptyl;
2-Chlorethyl, 2- und 3-Chlorpropyl, 2-, 3- und 4-Chlorbutyl, 2-Bromethyl, 2- und 3-Brompropyl und 2-, 3- und 4-Brombutyl;
2-Nitroethyl, 2- und 3-Nitropropyl und 2-, 3- und 4-Nitrobutyl;
Methoxy, Ethoxy, Propoxy, Isopropoxy, Butoxy, Isobutoxy, sec.-Butoxy, tert.-Butoxy, Pentoxy, Isopentoxy, Neopentoxy, tert.-Pentoxy und Hexoxy;
Methylthio, Ethylthio, Propylthio, Isopropylthio, Butylthio, Isobutylthio, sec.-Butylthio, tert.-Butylthio, Pentylthio, Isopentylthio, Neopentylthio, tert.-Pentylthio und Hexylthio;
Ethinyl, 1- und 2-Propinyl, 1-, 2- und 3-Butinyl, 1-, 2-, 3- und 4-Pentinyl, 1-, 2-, 3-, 4- und 5-Hexinyl, 1-, 2-, 3-, 4-, 5-, 6-, 7-, 8- und 9-Decinyl, 1-, 2-, 3-, 4-, 5-, 6-, 7-, 8-, 9-, 10- und 11-Dodecinyl und 1-, 2-, 3-, 4-, 5-, 6-, 7-, 8-, 9-, 10-, 11-, 12-, 13-, 14-, 15-, 16- und 17-Octadecinyl;
Ethenyl, 1- und 2-Propenyl, 1-, 2- und 3-Butenyl, 1-,2-,3- und 4-Pentenyl, 1-, 2-, 3-, 4- und 5-Hexenyl, 1-, 2-, 3-, 4-, 5-, 6-, 7-, 8- und 9-Decenyl, 1-, 2-, 3-, 4-, 5-, 6-, 7-, 8-, 9-, 10- und 11-Dodecenyl und 1-, 2-, 3-, 4-, 5-, 6-, 7-, 8-, 9-, 10-, 11-, 12-, 13-, 14-, 15-, 16- und 17-Octadecenyl;
Methylamino, Ethylamino, Propylamino, Isopryplamino, Butylamino, Isobutylamino, Pentylamino, Hexylamino, Dimethylamino, Methylethylamino, Diethylamino, Dipropylamino, Diisopropylamino, Dibutylamino, Diisobutylamino, Dipentylamino, Dihexylamino, Dicyclopentylamino, Dicyclohexytamino, Dicycloheptylamino, Diphenylamino und Dibenzylamino;
Formylamino, Acetylamino, Propionylamino und Benzoylamino;
Carbamoyl, Methylaminocarbonyl, Ethylaminocarbonyl, Propylaminocarbonyl, Butylaminocarbonyl, Pentylaminocarbonyl, Hexylaminocarbonyl, Heptylaminocarbonyl, Octylaminocarbonyl, Nonylaminocarbonyl, Decylaminocarbonyl und Phenylaminocarbonyl;
Aminosulfonyl, N,N-Dimethylaminosulfonyl, N,N-Diethylaminosulfonyl, N-Methyl-N-ethylaminosulfonyl, N-Methyl-N-dodecylaminosulfonyl, N-Dodecylaminosulfonyl, (N,N-Dimethylamino)ethylaminosulfonyl, N,N-(Propoxyethyl)dodecylaminosulfonyl, N,N-Diphenylaminosulfonyl, N,N-(4-tert.-Butylphenyl)octadecylaminosulfonyl und N,N-Bis(4-Chlorphenyl)aminosulfonyl;
Methoxycarbonyl, Ethoxycarbonyl, Propoxycarbonyl, Isopropoxycarbonyl, Hexoxycarbonyl, Dodecyloxycarbonyl, Octadecyloxycarbonyl, Phenoxycarbonyl, (4-tert.-Butyl-phenoxy)carbonyl und (4-Chlorphenoxy)carbonyl;
Methoxysulfonyl, Ethoxysulfonyl, Propoxysulfonyl, Isopropoxysulfonyl, Butoxysulfonyl, Isobutoxysulfonyl, tert.-Butoxysulfonyl, Hexoxysulfonyl, Dodecyloxysulfonyl, Octadecyloxysulfonyl, Phenoxysulfonyl, 1- und 2-Naphthyloxysulfonyl, (4-tert.-Butylphenoxy)-sulfonyl und (4-Chlorphenoxy)sulfonyl;
Diphenylphosphino, Di-(o-tolyl)phosphino und Diphenylphosphinoxido;
Chlor, Brom und Iod;
Phenylazo, 2-Napthylazo, 2-Pyridylazo und 2-Pyrimidylazo;
Cyclopropyl, Cyclobutyl, Cyclopentyl, 2- und 3-Methylcyclopentyl, 2- und 3-Ethytcyclopentyl, Cyclohexyl, 2-, 3- und 4-Methylcyclohexyl, 2-, 3- und 4-Ethyfcyclohexyl, 3- und 4-Propylcyclohexyl, 3- und 4-Isopropylcyclohexyl, 3- und 4-Butylcyclohexyl, 3- und 4-sec.-Butylcyclohexyl, 3- und 4-tert.-Butylcyclohexyl, Cycloheptyl, 2-, 3- und 4-Methylcycloheptyl, 2-, 3- und 4-Ethylcycloheptyl, 3- und 4-Propylcycloheptyl, 3- und 4-Isopropylcycloheptyl, 3- und 4-Butylcycloheptyl, 3- und 4-sec.-Butylcycloheptyl, 3- und 4-tert.-Butylcycloheptyl, Cyclooctyl, 2-, 3-, 4- und 5-Methylcyclooctyl, 2-, 3-, 4- und 5-Ethylcyclooctyl und 3-, 4- und 5-Propylcyclooctyl; 3- und 4-Hydroxycyclohexyl, 3- und 4- Nitrocyclohexyl und 3- und 4-Chlorcyclohexyl;
1-, 2- und 3-Cyclopentenyl, 1-,2-,3- und 4-Cyclohexenyl, 1-, 2- und 3-Cycloheptenyl und 1-, 2-, 3- und 4-Cyclooctenyl;
2-Dioxanyl, 1-Morpholinyl, 1-Thiomorpholinyl, 2- und 3-Tetrahydrofuryl, 1-, 2- und 3-Pyrrolidinyl, 1-Piperazyl, 1-Diketopiperazyl und 1-, 2-, 3- und 4-Piperidyl;
Phenyl, 2-Naphthyl, 2- und 3-Pyrryl, 2-, 3- und 4-Pyridyl, 2-, 4- und 5-Pyrimidyl, 3-, 4- und 5-Pyrazolyl, 2-, 4- und 5-Imidazolyl, 2-, 4- und 5-Thiazolyl, 3-(1,2,4-Triazyl), 2-(1,3,5-Triazyl), 6-Chinaldyl, 3-, 5-, 6- und 8-Chinolinyl, 2-Benzoxazolyl, 2-Benzothiazolyl, 5-Benzothiadiazolyl, 2- und 5-Benzimidazolyl und 1- und 5-Isochinolyl;
1-, 2-, 3-, 4-, 5-, 6- und 7-Indolyl, 1-, 2-, 3-, 4-, 5-, 6- und 7-Isoindolyl, 5-(4-Methylisoindolyl), 5-(4-Phenylisoindolyl), 1-, 2-, 4-, 6-, 7- und 8-(1,2,3,4-Tetrahydroisochinolinyl), 3-(5-Phenyl)-(1,2,3,4-tetrahydroisochinolinyl), 5-(3-Dodecyl-(1,2,3,4-tetrahydroisochinolinyl), 1-, 2-, 3-, 4-, 5-, 6-, 7- und 8-(1,2,3,4-Tetrahydrochinolinyl) und 2-, 3-, 4-, 5-, 6-, 7- und 8-Chromanyl, 2-, 4- und 7-Chinolinyl, 2-(4-Phenylchinolinyl) und 2-(5-Ethylchinolinyl);
2-, 3- und 4-Methylphenyl, 2,4-, 3,5- und 2,6-Dimethylphenyl, 2,4,6-Trimethyfphenyl, 2-, 3- und 4-Ethylphenyl, 2,4-, 3,5- und 2,6-Diethylphenyl, 2,4,6-Triethylphenyl, 2-, 3- und 4-Propylphenyl, 2,4-, 3,5- und 2,6-Dipropylphenyl, 2,4,6-Tripropylphenyl, 2-, 3- und 4-Isopropylphenyl, 2,4-, 3,5- und 2,6-Diisopropylphenyl, 2,4,6-Triisopropylphenyl, 2-, 3-und 4-Butylphenyl, 2,4-, 3,5- und 2,6-Dibutylphenyl, 2,4,6-Tributylphenyl, 2-, 3- und 4-Isobutylphenyl, 2,4-, 3,5- und 2,6-Diisobutylphenyl, 2,4,6-Triisobutylphenyl, 2-, 3- und 4-sec.-Butylphenyl, 2,4-, 3,5- und 2,6-Di-sec.-butylphenyl und 2,4,6-Tri-sec.-butylphenyl; 2-, 3- und 4-Methoxyphenyl, 2,4-, 3,5- und 2,6-Dimethoxyphenyl, 2,4,6-Trimethoxyphenyl, 2-, 3- und 4-Ethoxyphenyl, 2,4-, 3,5- und 2,6-Diethoxyphenyl, 2,4,6-Triethoxyphenyl, 2-, 3- und 4-Propoxyphenyl, 2,4-, 3,5- und 2,6-Dipropoxyphenyl, 2-, 3-und 4-Isopropoxyphenyl, 2,4- und 2,6-Diisopropoxyphenyl und 2-, 3- und 4-Butoxyphenyl; 2-, 3- und 4-Chlorphenyl und 2,4-, 3,5- und 2,6-Dichlorphenyl; 2-, 3- und 4-Hydroxyphenyl und 2,4-, 3,5- und 2,6-Dihydroxyphenyl; 2-, 3- und 4-Cyanophenyl; 3-und 4-Carboxyphenyl; 3- und 4-Carboxamidophenyl, 3- und 4-N-Methylcarboxamidophenyl und 3- und 4-N-Ethylcarboxamidophenyl; 3- und 4-Acetylaminophenyl, 3- und 4-Propionylaminophenyl und 3- und 4-Buturylaminophenyl; 3- und 4-N-Phenylaminophenyl, 3- und 4-N-(o-Tolyl)aminophenyl, 3- und 4-N-(m-Tolyl)aminophenyl und 3- und 4-(p-Tolyl)aminophenyl; 3- und 4-(2-Pyridyl)aminophenyl, 3- und 4-(3-Pyridyl)aminophenyl, 3- und 4-(4-Pyridyl)aminophenyl, 3- und 4-(2-Pyrimidyl)aminophenyl und 4-(4-Pyrimidyl)aminophenyl;
4-Phenylazophenyl, 4-(1-Naphthylazo)phenyl, 4-(2-Naphthylazo)phenyl, 4-(4-Naphthylazo)phenyl,4-(2-Pyrylazo)phenyl, 4-(3-Pyridylazo)phenyl, 4-(4-Pyridylazo)phenyl, 4-(2-Pyrimidylazo)phenyl, 4-(4-Pyrimidylazo)phenyl und 4-(5-Pyrimidylazo)phenyl;
Phenoxy, Phenylthio, 2-Naphthoxy, 2-Naphthylthio, 2-, 3- und 4-Pyridyloxy, 2-, 3- und 4-Pyridylthio, 2-, 4- und 5-Pyrimidyloxy und 2-, 4- und 5-Pyrimidylthio.
Die die erfindungsgemäßen Rylenderivate Ia kennzeichnenden Substituenten A können vorteilhaft durch Umsetzung der entsprechenden halogenierten (vorzugsweise der chlorierten oder vor allem bromierten) Rylenderivate mit ortho,ortho'-disubstituierten (Thio)Phenolen HA der allgemeinen Formel II 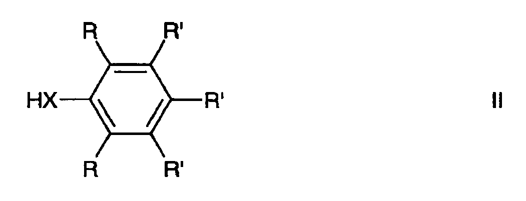 in das Rylengerüst eingeführt werden.

Diese Umsetzung wird zweckmäßig in Gegenwart einer Base und eines nichtnucleophilen Lösungsmittels vorgenommen.

Das (Thio)Phenol HA kann jedoch auch vorab in das (Thio)Phenolat überführt werden. In diesem Fall kann bei der nucleophilen Substitution auf die Anwesenheit der Base verzichtet werden.

Durch unvollständigen Halogenaustausch sind auf diese Weise auch Rylenderivate, die sowohl die Reste A als auch Halogenatome als Substituenten am Ringsystem tragen, zu erhalten.

Die nucleophile Substitution an den halogenierten Rylenderivaten bietet auch die Möglichkeit, Rylenderivate Ia herzustellen, die neben den Resten A andere zusätzliche Substituenten aufweisen.

So können Rylenderivate la, die sowohl die Reste A als auch weitere (Thio)Phenoxyreste Y, die nicht ortho,ortho'-disubstituiert sind, als Substituenten tragen, leicht durch An(thio)phenoxylierung mit dem einen (Thio)Phenol HA oder HY und ergänzende Umsetzung mit dem jeweils anderen (Thio)Phenol HY bzw. HA oder auch parallele Umsetzung mit beiden (Thio)Phenolen hergestellt werden.

Rylenderivate la, die als zusätzliche Substituenten cyclische Aminogruppen P aufweisen, sind analog zu erhalten, also durch An(thio)phenoxylierung mit dem ortho,ortho'-disubstituierten (Thio)Phenol HA und ergänzende Umsetzung mit einem Amin HP oder zunächst teilweise Umsetzung mit dem Amin HP und dann ergänzende (Thio)Phenoxy-lierung oder aber durch parallele Umsetzung des halogenierten Rylenderivats mit HA und HP.

Rylenderivate Ia, die Kombinationen von mindestens 3 verschiedenen Substituenten aufweisen, sind selbstverständlich entsprechend herstellbar.

Die Umsetzung der halogenierten Rylenderivate mit dem (Thio)Phenol HA ist im folgenden am Beispiel der Perylen-, Terrylen- und Quaterrylentetracarbonsäurediimide und -dicarbonsäuremonoimide (im folgenden "Rylencarbonsäureimide" genannt) genauer beschrieben.

Als nichtnucleophile Lösungsmittel eignen sich für diese Umsetzung vor allem polare aprotische Lösungsmittel, insbesondere aliphatische Carbonsäureamide (vorzugsweise N,N-Di-C₁-C₄-alkyl-C₁-C₄-carbonsäureamide) und Lactame, wie Dimethylformamid, Diethylformamid, Dimethylacetamid, Dimethylbutyramid und N-Methylpyrrolidon.

Als nichtnucleophile Lösungsmittel können auch unpolare aprotische Lösungsmittel eingesetzt werden, diese Lösungsmittel sind jedoch nicht bevorzugt. Beispielhaft seien aromatische Kohlenwasserstoffe, wie Benzol, Toluol und Xylol, genannt.

Selbstverständlich können auch Lösungsmittelmischungen verwendet werden.

Die Lösungsmittelmenge hängt von der Löslichkeit des halogenierten Rylenderivats ab. In der Regel werden 2 bis 200 ml, insbesondere 3 bis 150 ml Lösungsmittel je g halogeniertes Rylenderivat benötigt.

Als Base eignen sich vor allem anorganische und organische alkali- oder erdalkalimetallhaltige Basen, wobei die alkalimetallhaltigen Basen besonders geeignet sind. Beispiele für anorganische Basen sind Alkali- und Erdalkalimetallcarbonate und - hydro-gencarbonate, -hydroxide, -hydride und -amide, Beispiele für organische Basen sind Alkoholate (insbesondere die C₁-C₁₀-Alkoholate, vor allem tert.-C₄-C₆-Alkoholate), (Phenyl)Alkylamide (insbesondere die Bis(C₁-C₄-alkyl)amide) und Triphenylmethylmetallate auf Basis der Alkali- und Erdalkalimetalle. Bevorzugte Basen sind die Carbonate und Hydrogencarbonate, wobei die Carbonate besonders bevorzugt sind. Bevorzugte Alkalimetalle sind Lithium, Natrium, Kalium und Caesium, besonders geeignete Erdalkalimetalle sind Magnesium und Calcium.

Als Beispiele für die metallhaltigen Basen seien im einzelnen genannt: Lithiumcarbonat, Natriumcarbonat, Kaliumcarbonat und Caesiumcarbonat; Natriumhydrogencarbonat und Kaliumhydrogencarbonat; Lithiumhydroxid, Natriumhydroxid, Kaliumhydroxid und Caesiumhydroxid; Lithiumhydrid, Natriumhydrid und Kaliumhydrid; Lithiumamid, Natriumamid und Kaliumamid; Lithiummethylat, Natriummethylat, Kaliummethylat, Lithiumethylat, Natriumethylat, Kaliumethylat, Natriumisopropylat, Kaliumisopropylat, Natrium-tert.-butylat, Kalium-tert.-butylat, Lithium-(1,1-dimethyl)octylat, Natrium-(1,1-dimethyl)octylat und Kalium-(1,1-dimethyl)octylat; Lithiumdimethylamid, Lithiumdiethyl-amid, Lithiumdiisopropylamid, Natriumdiisopropylamid, Lithiumhexamethyldisilazid, Natriumhexamethyldisilazid, Kaliumhexamethyldisilazid, Triphenylmethyllithium, Triphenylmethylnatrium und Triphenylmethylkalium.

Neben diesen metallhaltigen Basen eignen sich auch rein organische stickstoffhaltige Basen.

Geeignete Beispiele hierfür sind Alkylamine, insbesondere Tri(C₂-C₆-alkyl)amine, wie Triethylamin, Tripropylamin und Tributylamin, Alkoholamine, insbesondere Mono-, Di- und Tri(C₂-C₄-alkohol)amine, wie Mono-, Di- und Triethanolamin, und heterocyclische Basen, wie Pyridin, 4-(N,N-Dimethylamino)pyridin, (4-Pyrrolidino)pyridin, N-Methylpiperidin, N-Methylpiperidon, N-Methylmorpholin, N-Methyl-2-pyrrolidon, Pyrimidin, Chinolin, Isochinolin, Chinaldin, Diazabicyclooctan (DABCO), Diazabicyclononen (DBN) und Diazabicycloundecen (DBU).

Selbstverständlich können auch Basenmischungen verwendet werden.

Ganz besonders bevorzugte Basen sind Lithiumcarbonat, Natriumcarbonat, Kaliumcarbonat und Caesiumcarbonat.

In der Regel werden mindestens 0,4 Äquivalente Base je mol (Thio)Phenol HA benötigt. Besonders geeignete Einsatzmengen betragen für die Metallbasen 0,4 bis 3, insbesondere 0,4 bis 1,2 Äquivalente je mol HA. Bei den rein organischen Basen liegt die Einsatzmenge bevorzugt bei 0,4 bis 10, besonders bevorzugt bei 0,4 bis 3 Äquivalenten je mol HA. Wird die organische Base gleichzeitig als Lösungsmittel eingesetzt, was insbesondere bei den heterocyclischen Basen der Fall sein kann, ist eine mengenmäßige Beschränkung selbstverständlich überflüssig.

Die Umsetzung kann in Gegenwart von Phasentransferkatalysatoren vorgenommen werden.

Als Phasentransferkatalysatoren sind vor allem quartäre Ammoniumsalze und Phosphoniumsalze, wie Tetra(C₁-C₁₈-alkyl)ammoniumhalogenide und -tetrafluoroborate, Benzyltri(C₁-C₁₈-alkyl)ammoniumhalogenide und -tetrafluoroborate und Tetra(C₁-C₁₈-alkyl)- und Tetraphenylphosphoniumhalogenide, und Kronenether geeignet. Bei den Halogeniden handelt es sich in der Regel um die Fluoride, Chloride, Bromide und Iodide, wobei die Chloride und Bromide bevorzugt sind. Besonders geeignete Beispiele sind im einzelnen: Tetraethylammoniumchlorid, Tetrabutylammoniumbromid, Tetrabutylammoniumiodid, Tetrabutylammoniumtetrafluoroborat und Benzyltriethylammmoniumchlorid; Tetrabutylphosphoniumbromid und Tetraphenylphosphoniumchlorid und -bromid; 18-Krone-6, 12-Krone-4 und 15-Krone-5.

Wird ein Phasentransferkatalysator verwendet, so liegt seine Einsatzmenge üblicherweise bei 0,4 bis 10, insbesondere 0,4 bis 3 Äquivalenten je mol (Thio)Phenol. In der Regel werden je mol auszutauschendes Halogenatom 1 bis 10 mol, vorzugsweise 1 bis 5 mol (Thio)Phenol HA eingesetzt. Soll nur ein teilweiser Ersatz der Halogenatome erfolgen, so empfiehlt es sich, die (Thio)Phenolmenge auf 1 bis 1,5, bevorzugt 1 bis 1,2 mol je mol auszutauschendes Halogenatom zu erniedrigen.

Die Reaktionstemperatur hängt von der Reaktivität des Substrats ab und liegt im Allgemeinen im Bereich von 30 bis 150°C.

Die Reaktionszeit beträgt üblicherweise 0,5 bis 96 h, insbesondere 2 bis 72 h.

Verfahrenstechnisch kann man auf unterschiedliche Weise vorgehen. Man kann die Reaktionspartner zunächst mischen und dann gemeinsam auf die Reaktionstemperatur erwärmen. Insbesondere zur Erzielung höherer Substitutionsgrade kann es von Vorteil sein, zunächst nur einen Teil der (Thio)Phenols HA und der Base vorzulegen und den Rest, gegebenenfalls nach Zwischenisolierung des in den reaktiveren Positionen teil(thio)phenoxylierten Produkt, erst später zuzugeben.

Die Isolierung der erhaltenen Rylencarbonsäureimide Ia kann man bei Einsatz anorganischer Basen wie folgt vornehmen:

Man kann die angefallenen anorganischen Salze zunächst abfiltrieren, das Rylencarbonsäureimid Ia dann durch Zugabe von aliphatischen Alkoholen, wie Methanol, Ethanol, Isopropanol, Butanol oder Ethylenglykolmonobutylether, von Wasser oder von Wasser/Alkohol-Gemischen oder durch Verdampfen des Lösungsmittels ausfällen und abschließend abfiltrieren.

Man kann die anorganischen Salze jedoch auch zusammen mit dem ausgefällten Rylencarbonsäureimid Ia abfiltrieren und durch Waschen mit Wasser und/oder verdünnten anorganischen Säuren, wie Salzsäure oder Schwefelsäure, auswaschen.

Gewünschtenfalls können die erhaltenen Rylencarbonsäureimide Ia zur zusätzlichen Reinigung einer Filtration oder Säulenchromatographie an Kieselgel unterzogen werden. Als Eluens eignen sich dabei insbesondere halogenierte aliphatische Kohlenwasserstoffe, wie Methylenchlorid und Chloroform, aliphatische und aromatische Kohlenwasserstoffe, wie Cyclohexan, Petrolether, Benzol, Toluol und Xylol, aliphatische Alkohole, wie Methanol und Ethanol, und aliphatische Carbonsäureester, wie Essigsäureethylester, die vorzugsweise in Form von Mischungen eingesetzt werden.

Bei nur teil(thio)phenoxylierten Rylencarbonsäureimiden Ia kann man das noch enthaltene Halogen gewünschtenfalls entfernen. Dies kann vorteilhaft, wie in der WO-A-02/76988 beschrieben, durch eine übergangsmetallkatalysierte reduktive Enthalogenierung in Gegenwart eines unter den Reaktionsbedingungen inerten Lösungsmittels oder baseninduziert in Gegenwart eines inerten stickstoffbasischen oder aromatischen Lösungsmittels geschehen.

Die Enthalogenierung kann vor der Isolierung des Reaktionsprodukts in dem bei der (Thio)Phenoxylierung anfallenden Reaktionsgemisch vorgenommen werden. Gewünschtenfalls kann das Reaktionsprodukt jedoch auch erst zwischenisoliert und gegebenenfalls zusätzlich gereinigt werden. Die baseninduzierte Enthalogenierung auch direkt mit der (Thio)Phenoxylierung verbunden werden, indem von vornherein starke Basen in größerer Menge eingesetzt werden und das Reaktionsgemisch nach erfolgter (Thio)Phen-oxylierung auf höhere Temperatur erhitzt wird.

Für die reduktive Enthalogenierung eignen sich als Reduktionsmittel vor allem komplexe Hydride, insbesondere Borhydride, wie Natriumborhydrid, und elementarer Wasserstoff.

Die Menge an Reduktionsmittel beträgt im Falle der komplexen Hydride in der Regel 1 bis 8, vorzugsweise 2 bis 5 Äquivalente je mol zu eliminierendes Halogenatom.

Bei der Reduktion mit Wasserstoff wird so viel Wasserstoff zugesetzt, bis die Reaktion abgeschlossen ist. Dabei kann bei einem Wasserstoffdruck von 1 bis 100 bar gearbeitet werden, in der Regel wird ein Wasserstoffdruck um 1 bar jedoch ausreichen.

Bei Verwendung von komplexen Hydriden als Reduktionsmittel eignen sich als Übergangsmetallkatalysatoren insbesondere Palladiumverbindungen, wie Palladiumacetat, Dichloro(1,5-cyclooctadien)palladium(II), Dichloro[1,1'-bis(diphenylphosphino)ferro-cen]palladium(II), Tri(dibenzylidenaceton)dipalladium(0), Tetrakis(triphenylphosphin)-palladium(0) und Tetrakis(tris-o-tolylphosphin)palladium(0)

Bei Verwendung von Wasserstoff als Reduktionsmittel können ebenfalls die oben genannten Übergangsmetallkatalysatoren eingesetzt werden, bevorzugt wird jedoch Palladium auf Aktivkohle verwendet.

In der Regel werden 0,5 bis 10, vorzugsweise 0,5 bis 5 mol-% Katalysator je mol zu eliminierendes Halogenatom eingesetzt.

Als inertes Lösungsmittel eignen sich insbesondere aliphatische Carbonsäurenitrile, wie Acetonitril und Propionitril, aliphatische Carbonsäureamide, wie N,N-Dimethylformamid und N,N-Dimethylacetamid, und Lactame, wie N-Methylpyrrolidon.

Die Lösungsmittelmenge beträgt in der Regel 10 bis100 ml Lösungsmittel je g zu enthalogenierendes Rylencarbonsäureimid la.

Bei der reduktiven Enthalogenierung mit komplexen Hydriden empfiehlt es sich, unter Schutzgas zu arbeiten.

Die Reaktionstemperatur liegt bei Verwendung der komplexen Hydride üblicherweise im Bereich von 20 bis 100°C und bei der Reduktion mit Wasserstoff bei 20 bis 50°C.

Die Reaktionszeit beträgt in der Regel 5 bis 100 h, bevorzugt 12 bis 60 h.

Die reduktive Enthalogenierung kann direkt im Anschluß an die (Thio)Phenoxylierung vorgenommen werden, indem man das Reaktionsgemisch auf Raumtemperatur abkühlt, das Reduktionsmittel und den Katalysator zusetzt und die Mischung dann auf die gewünschte Reaktionstemperatur erhitzt. Gewünschtenfalls kann man das (thio)phen-oxylierte Produkt jedoch auch erst zwischenisolieren und gegebenenfalls zusätzlich reinigen.

Für die baseninduzierte Enthalogenierung eignen sich als Basen insbesondere Alkalimetallhydroxide, wie Kalium- und Natriumhydroxid, Alkalimetallcarbonate, wie Kalium-, Natrium- und Caesiumcarbonat, Alkalimetallalkoholate sekundärer und tertiärer Alkohole, wie Lithium-, Natrium- und Kaliumisopropylat und -tert.-butylat, sowie sterisch gehinderte Stickstoffbasen, wie DABCO, DBN und DBU.

In der Regel werden 1 bis 3, vorzugsweise 1 bis 1,5 Äquivalente Base je mol zu eliminierendes Halogenatom eingesetzt.

Als Lösungsmittel können die gleichen Lösungsmittel wie bei der (Thio)Phenoxylierung verwendet werden.

Die Lösungsmittelmenge beträgt zweckmäßigerweise 2 bis 200 ml, insbesondere 3 bis 100 ml je g zu enthalogenierendes Rylencarbonsäureimid la.

Die Reaktionstemperatur liegt üblicherweise im Bereich von 50 bis 200°C, vorzugsweise bei 60 bis 130°C.

Es empfiehlt sich, die Enthalogenierung unter Schutzgas vorzunehmen.

Die baseninduzierte Enthalogenierung ist im allgemeinen in 5 bis 72 h, vor allem in 10 bis 48 h beendet.

Zur Herstellung aller erfindungsgemäßen funktionalisierten Perylen-, Terrylen- und Quaterrylenderivate Ia geht man vorzugsweise von den entsprechenden halogenierten Rylentetracarbonsäurediimiden aus, die z.B. in den WO-A-97/22607, 03/104232 und 96/22332 beschrieben sind.

Nach der (Thio)Phenoxylierung können die Dimide dann gewünschtenfalls durch Verseifung in die Rylentetracarbonsäuredianhydride Ia oder die Rylentetracarbonsäuremonoimidmonoanhydride Ia überführt werden.

Die Rylentetracarbonsäuremonoimidmonoanhydride Ia können zu den Rylendicarbonsäureimiden Ia decarboxyliert werden, aus denen wiederum durch Verseifung die entsprechenden Rylendicarbonsäureanhydride Ia zugänglich sind.

Selbstverständlich können die Rylendicarbonsäureimide Ia auch, wie vorstehend beschrieben, durch (Thio)Phenoxylierung der halogenierten Rylendicarbonsäureimide hergestellt werden.

Die Rylentetracarbonsäuredianhydride Ia können durch Decarboxylierung in die nichtfunktionalisierten Rylene Ia oder durch Umsetzung mit aromatischen Diaminen H₂N- E- NH₂ in die entsprechenden Di- und Monokondensationsprodukte überführt werden.

Aus den Rylentetracarbonsäuremonoimidmonoanhydriden Ia sind schließlich durch Umsetzung mit aromatischen Diaminen H₂N- E- NH₂ auch die entsprechenden Rylentetracarbonsäuremonoimidsemikondensationsprodukte erhältlich.

Die Folge dieser Umfunktionalisierungen ist am Beispiel der Terrylen- und Quaterrylenderivate in der älteren deutschen Patentanmeldung 10 2005 021 362.6 beschrieben.

Pentarylen- und Hexarylentetracarbonsäurediimide sind schließlich durch Kupplungsreaktion von mit den Resten A (und gewünschtenfalls mit den Resten Y) substituierten Perylen- und Terrylendicarbonsäureimiden bzw. durch Homokupplung entsprechend substituierter Terrylendicarbonsäureimide zugänglich. Diese Kupplungsreaktionen sind in der älteren deutschen Patentanmeldung 10 2005 018 241.0 näher beschrieben.

Die erfindungsgemäßen Rylenderivate Ia zeichnen sich durch steile Absorptionsbanden aus. Sie weisen daher besonders brillante Absorptions- bzw. Fluoreszenzfarbtöne auf. Bei fluoreszierenden erfindungsgemäßen Rylenderivaten la ist außerdem die Fluoreszenzquantenausbeute erhöht. Gleichzeitig sind die Absorptionsmaxima langwellig gegenüber den jeweils entsprechenden, nicht durch die Reste A substituierten Individuen verschoben, was für viele Anwendungen eine bessere Ausnutzung des eingestrahlten Lichts zur Folge hat.

Die erfindungsgemäßen Rylenderivate Ia können problemlos in organische und anorganische Materialien eingearbeitet werden und eignen sich daher für eine ganze Reihe von Anwendungszwecken, von denen einige im folgenden beispielhaft aufgeführt werden.

Sie können generell zur Einfärbung von Lacken, Druckfarben und Kunststoffen eingesetzt werden.

Die Perylen- und Terrylenderivate la absorbieren im sichtbaren Bereich des elektromagnetischen Spektrums und zeichnen sich dabei insbesondere durch ihre Fluoreszenz aus, die im Fall der Perylenderivate Ia im sichtbaren Bereich des elektromagnetischen Spektrums liegt.

Die höheren Rylenderivate Ia sind insbesondere aufgrund ihres Absorptionsvermögens im nahinfraroten Bereich des elektromagnetischen Spektrums von Interesse.

Die erfindungsgemäßen Rylenderivate Ia können zur Herstellung elektromagnetische Strahlung absorbierender und/oder emittierender wäßriger Polymerisatdispersionen verwendet werden. Fluoreszierende Polymerisatdispersionen ergeben sich bei den Terrylenderivaten Ia und insbesondere bei den Perylenderivaten Ia, während bei den höheren Homologen im nahinfraroten Bereich des elektromagnetischen Spektrums absorbierende Polymerisatdispersionen resultieren.

Weiterhin eignen sich insbesondere die höheren Ryfenderivate Ia aufgrund ihrer ausgeprägten Absorption im nahinfraroten Bereich des elektromagnetischen Spektrums zur Erzeugung für das menschliche Auge nicht sichtbarer, Infrarotlicht absorbierender Markierungen und Beschriftungen, als Infrarotabsorber für das Wärmemanagement und als IR-Laserstrahlenabsorbierende Materialien beim Schweißbehandeln von Kunststoffteilen. Diese Anwendungen sind z.B. in der DE-A-10 2004 018 547 und den WO-A-02177081 und 04/05427 näher beschrieben.

Die höheren Rylenderivate Ia können vorteilhaft auch zur Lasermarkierung und Laserbeschriftung eingesetzt werden. Hierbei bewirkt das durch die Rylenderivate la absorbierte Laserlicht eine Erwärmung des Kunststoffs, die zur seiner Aufschäumung oder der Umwandlung eines zusätzlich enthaltenen Farbmittels führt und auf diese Weise eine Markierung bzw. Beschriftung ergibt.

Außerdem können die erfindungsgemäßen Rylenderivate Ia als Halbleiter in der organischen Elektronik eingesetzt werden. Beispiele für einzelne auf diesem Gebiet liegenden Anwendungen sind Feldeffekttransistoren und die Elektrophotographie.

Die erfindungsgemäßen Rylenderivate Ia sind auch als Filter oder Emitter für Display-Anwendungen einsetzbar. Dabei sind die im sichtbaren Bereich absorbierenden, fluoreszierenden Rylenderivate Ia, vor allem also die Perylenderivate Ia, als absorbierende Colorfilter oder als fluoreszierende Emitter für LCD- und OLED-Displays von Interesse, während die im NIR absorbierenden höheren Rylenderivate I insbesondere als Schutzfilter vor NIR-Strahlung fungieren können.

Schließlich können die Rylenderivate Ia auch als Emitter in Chemilumineszenzanwendungen Anwendung finden. Hier sind wiederum die Fluoreszenzfarbmittel auf Perylen- und Terrylenbasis besonders geeignet.

Die Fluoreszenzfarbmittel auf Perylen- und Terrylenbasis sind außerdem als Markierungsgruppen in Nachweisverfahren, insbesondere in diagnostischen und analytischen Verfahren an biologischen Proben, einschließlich lebender Zellen, von Interesse.

Nicht zuletzt können die efindungsgemäßen Rylenderivate Ia, insbesondere die Perylen- und Terrylenderivate Ia, auch als Aktivkomponenten in der Photovoltaik zum Einsatz kommen.

### Beispiele

### Beispiel 1: N,N'-Bis(2,6-diisopropylphenyl)-1,7- und -1,6-bis(2,6-diisopropylphenoxy)-peryten-3.4:9,10-tetracarbonsäurediimid (la1)

Zu einer Mischung von 2,6 g (3 mmol) eines Gemisches von N,N'-Bis(2,6-diisopropylphenyl)-1,7- und -1,6-dibromperylen-3,4:9,10-tetracarbonsäurediimid (Isomerenverhältnis 75 : 25) und 1,39 g (7,5 mmol) 2,6-Diisopropylphenol in 150 ml N-Methylpyrrolidon wurden 1,24 g (9 mmol) Kaliumcarbonat zugegeben. Dann wurde die Mischung auf 95°C erhitzt und 4 h bei dieser Temperatur gerührt. Nach Abkühlen auf Raumtemperatur wurden langsam 150 ml Wasser zugegeben. Nach einstündigem Nachrühren wurde das ausgefällte Produkt abfiltriert, nacheinander portionsweise mit insgesamt 300 ml 10 gew.-%iger Schwefelsäure, mit Wasser und mit wenig Ethanol gewaschen und im Vakuum getrocknet.

Es wurden 2,4 g (75%) des Rylenderivatgemisches la1 in Form eines violetten Feststoffs erhalten, der zur weiteren Reinigung einer Säulenchromatographie an Kieselgel mit Toluol als Eluens unterzogen wurde.
Absorption: (max (CH2Cl2) = 557 nm;
Emission: (max (CH2Cl2) = 575 nm.

Das UV-Spektrum von la1 zeigt im Vergleich zu dem UV-Spektrum eines Gemisches von N,N'-Bis(2,6-diisopropylphenyl)-1,7- und -1,6-bis(4-tert.-octylphenoxy)perylen-3,4:9.10-tetracarbonsäurediimid (V1) eine deutlich steilere Absorptionsbande mit langwellig verschobenem Absorptionsmaximum (V1: (max (Absorption) (CH2Cl2) = 543 nm).

Die Rylenderivatgemische la1 und V1 wurden jeweils 0,02 gew.-%ig in PMMA eingearbeitet und zu einem Spritzling von 5,5 x 5,5 cm Kantenlänge verarbeitet.

Der das Rylenderivatgemisch la1 enthaltende Spritzling wies eine deutlich höhere Kantenfluoreszenz und einen deutlich brillanteren blauen Farbton auf.

### Beispiel 2: N,N'-Bis(2,6-diisopropylphenyl)-1,6,9,14-tetra(2,6-diisopropylphenoxy)-terrylen-3,4:11,12-tetracarbonsäurediimid (la2)

Zu einer Mischung von 3,45 g (3 mmol) N,N'-Bis(2,6-diisopropylphenyl)-1,6,9,14-tetrabromterrylen-3,4:11,12-tetracarbonsäurediimid und 2,8 g (15 mmol) 2,6-Diisopropylphenol in 200 ml N-Methylpyrrolidon wurden 2,48 g (18 mmol) Kaliumcarbonat zugegeben. Dann wurde die Mischung auf 80°C erhitzt und 5 h bei dieser Temperatur gerührt. Nach Abkühlen auf Raumtemperatur wurden 50 ml Wasser zugegeben. Nach zehnstündigem Nachrühren wurde das ausgefällte Produkt abfiltriert, zunächst portionsweise mit insgesamt 400 ml 10 gew.-%iger Schwefelsäure und anschließen mit Wasser neutral gewaschen und im Vakuum getrocknet.

Es wurden 4,25 g (92%) des Rylenderivats la2 in Form eines grünen Feststoffs erhalten, der zur weiteren Reinigung einer Säulenchromatographie an Kieselgel mit Toluol als Eluens unterzogen wurde.
Rf-Wert (Petrolether/Essigester 8:1) = 0,8;
Absorption: (max (CH2Cl2) = 694 nm; Massenextinktion E = 90 l g-1 cm-1;
Emission: λ ₘₐₓ (CH₂Cl₂) = 720 nm.

### Beispiel 3: N,N'-Bis(2,6-diisopropylphenyl)-1,6,11,16-tetra(2,6-diisopropylphenoxy)-quaterrylen-3,4:13,14-tetracarbonsäurediimid (la3)

Zu einer Mischung von 12,9 g (9 mmol) eines etwa gleichteiligen Gemisches von N,N'-Bis(2,6-diisopropylphenyl)-1,6,8,11,16,18- und -1,6,8,11,16,19-hexabromquaterrylen-3,4:13,14-tetracarbonsäurediimid und 8,4 g (45 mmol) 2,6-Diisopropylphenol in 600 ml N-Methylpyrrolidon wurden 2,52 g (18 mmol) Kaliumcarbonat in 4 h zugegeben. Dann wurde die Mischung zunächst auf 100°C erhitzt und 2 h bei dieser Temperatur gerührt, dann 2 h auf 110°C und abschließend 2 h auf 120°C erhitzt. Bei dieser Temperatur wurden weitere 2,0 g (11 mmol) 2,6-Diisopropylphenol und 0,63 g (4,5 mmol) Kaliumcarbonat zugegeben. Nach weiterem fünfstündigen Rühren bei 120°C wurde die Mischung auf Raumtemperatur abgekühlt.

Zur Enthalogenierung der hierbei als Hauptkomponente angefallenen Gemisches aus N,N'-Bis(2,6-diisopropylphenyl)-1,6,11,16-tetra(2,6-diisopropylphenoxy)-8,18- und -8,19-dibromquaterrylen-3,4:13,14-tetracarbonsäurediimid wurde das erhaltene Reaktionsgemisch mit 1,98 g (52 mmol) Natriumborhydrid und 0,12 g (0,1 mmol) Tetrakis(triphenylphosphin)palladium(0) versetzt und 13 h auf 70°C erhitzt.

Nach Abkühlen auf Raumtemperatur wurde das Produkt durch langsame Zugabe von zunächst 300 ml Wasser und dann 100 ml 10 gew.-%iger Schwefelsäure ausgefällt, abfiltriert, zunächst portionsweise mit insgesamt 400 ml 10 gew.-%iger Schwefelsäure und dann mit Wasser neutral gewaschen und im Vakuum getrocknet.

Es wurden 12,8 g (78%) des Rylenderivats Ia3 in Form eines grünen Feststoffs erhalten, der zur weiteren Reinigung einer Säulenchromatographie an Kieselgel mit Toluol als Eluens unterzogen wurde.
R_{f}-Wert (Petrolether/Essigester 8:1) = 0,5;
Absorption: λₘₐₓ (CH₂Cl₂) = 802 nm; Massenextinktion E = 98 I g⁻¹ cm⁻¹.

Das UV-Spektrum von Ia3 zeigt im Vergleich zu dem UV-Spektrum von N,N'-Bis(2,6-diisopropylphenyl)-1,6,11,16-tetra(4-tert.-octylphenoxy)quaterrylen-3,4:13,14-tetracarbonsäurediimid (V3) eine steilere bathochrom verschobene Absorptionsbande und geringere Restabsorption im sichtbaren Bereich (V3: (max (Absorption) (CH2Cl2) = 778 nm).

### Beispiel 4: N,N'-Bis(2,6-diisopropylphenyl)-1,6,11,16-tetra(2,6-diisopropylphenoxy)-8,18- und-8,19-dibromquaterrylen-3,4:13,14-tetracarbonsäurediimid (Ia4)

Eine Mischung von 18,0 g (13 mmol) eines etwa gleichteiligen Gemisches von N,N'-Bis(2,6-diisopropylphenyl)-1,6,8,11,16,18- und -1,6,8,11,16,19-hexabromquaterrylen-3,4:13,14-tetracarbonsäurediimid, 640 ml N-Methylpyrrolidon, 9,18 g (52 mmol) 2,6-Diisopropylphenol und 4,34 g (31 mmol) Kaliumcarbonat wurde auf 80°C erhitzt und 27 h bei dieser Temperatur gerührt.

Nach Abkühlen auf Raumtemperatur wurde das Reaktionsgemisch auf 2 l 10 gew.-%-ige Schwefelsäure gefällt. Das Produkt wurde abfiltriert, zunächst portionsweise mit insgesamt 500 ml 10 gew.-%iger Schwefelsäure und dann mit Ethanol gewaschen und im Vakuum getrocknet.

Es wurden 22,1 (quantitative Umsetzung) des Rylenderivatgemischs Ia4 in Form eines grünen Feststoffs erhalten, der zur weiteren Reinigung einer Säulenchromatographie an Kieselgel mit einem Toluol/Methylenchlorid-Gemisch (9:1) als Eluens unterzogen wurde.

### Beispiel 5: N-(2,6-Diisopropylphenyl)-9-(2,6-diisopropylphenoxy)perylen-3,4-dicarbonsäureimid (Ia5)

Eine Mischung von 7,0 g (25 mmol) N-(2,6-Diisopropylphenyl)-9-bromperylen-3,4-dicarbonsäureimid, 2,3 g (25 mmol) 2,6-Diisopropylphenol, 100 ml N-Methylpyrrolidon und 3,45 g (50 mmol) Kaliumcarbonat wurde 3 h auf 100°C erhitzt. Nach Abkühlen auf Raumtemperatur wurden 17 ml Wasser zugegeben. Das ausgefällte Produkt wurde abfiltriert, zunächst mit 135 ml 5 gew.-%iger Schwefelsäure und dann mit Wasser neutral gewaschen und im Vakuum getrocknet.

Es wurden 7,7 g (50%) des Rylenderivats Ia5 in Form eines violetten Feststoffs erhalten, der zur weiteren Reinigung mit einem Toluol/Essigester-Gemisch (60:1) als Eluens über Kieselgel filtriert wurde.
Rf-Wert (Toluol/Essigester 60:1) = 0,24.

### Beispiel 6: N,N'-Bis(2,6-diisopropylphenyl)-1,6,9,14-tetra(2,6-dimethylthiophenoxy)-terrylen-3,4:11,12-tetracarbonsäurediimid (Ia6)

Eine Lösung von 0,8 g (0,7 mmol) N,N'-Bis(2,6-diisopropylphenyl)-1,6,9,14-tetrabromterrylen-3,4:11,12-tetracarbonsäurediimid in 50 ml N-Methylpyrrolidon wurde mit 0,4 g (2,8 mmol) 2,6-Dimethylthiophenol und 0,57 g (7 mmol) Kaliumcarbonat versetzt und auf 40°C erhitzt. Nach vierstündigem Rühren bei 40°C wurden 80 ml 5 gew.-%ige Schwefelsäure zugegeben. Das ausgefällte Produkt wurde abfiltriert, nacheinander mit Wasser, 2 gew.-%iger Natronlauge, Wasser und Ethanol gewaschen und im Vakuum getrocknet. Es wurden 1,1 g (quantitative Umsetzung) Ia6 in Form eines grünen Feststoffs erhalten, der zur weiteren Reinigung einer Säulenchromatographie an Kieselgel mit Toluol als Eluens unterzogen wurde.
R_{f}-Wert (Toluol/Essigester 10:1) = 0,69;
Absorption: λ ₘₐₓ (CH₂Cl₂) = 702 nm; Massenextinktion E = 60 l g⁻¹ cm⁻¹;
Emission: λ ₘₐₓ (CH₂Cl₂) = 757 nm; schwache Fluoreszenz.

### Beispiel 7: N,N'-Bis(2,6-diisopropylphenyl)-1,6,11,16-tetra(2,6-diisopropylphenoxy)-8,18- und-8,19-dipiperidylquaterrylen-3,4:13,14-tetracarbonsäurediimid (Ia7)

Eine Mischung aus 0,6 g (0,3 mmol) des in Beispiel 4 erhaltenen, durch Säulenchromatographie gereinigten Rylenderivatgemischs und 5,3 g (6,2 ml, 62 mmol) Piperidin wurde 120 h in einem auf 90°C temperierten Ölbad erhitzt. Nach Abkühlen auf Raumtemperatur wurde das Reaktionsgemisch auf eine Mischung aus 30 ml Methanol und
30 ml Wasser gefällt. Das Produkt wurde abfiltriert, mit 11 ml Methanol gewaschen, 3 h in 15 ml 15 gew.-%iger Schwefelsäure aufgerührt, dann erneut abfiltriert und mit Wasser neutral gewaschen.

Es wurden 0,54 g (90%) des Rylenderivatgemischs Ia7 in Form eines grünen Feststoffs erhalten, der gemäß Maldi-MS auch untergeordnete Mengen Tetraphenoxymonopiperidylmonobromderivat und Tetraphenoxymonopiperidylderivat enthielt.
Absorption: λₘₐₓ (N-Methylpyrrolidon) = 822 nm; Massenextinktion E = 34,4 l g⁻¹ cm⁻¹.

## Patentansprüche

1. Rylenderivate der allgemeinen Formel la 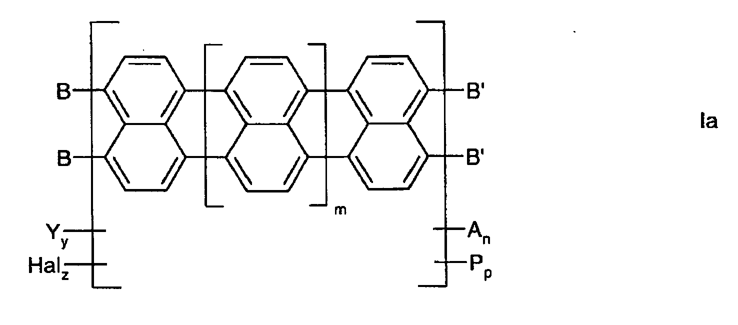 in der die Variablen folgende Bedeutung haben:
B miteinander unter Ausbildung eines Sechsrings verbunden zu einem Rest der Formel (a) oder (b) 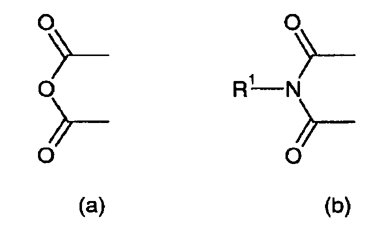 beide Wasserstoff oder ein Rest -COOM oder einer der beiden Reste ein Rest A oder Hal und der andere Rest Wasserstoff;
B' unabhängig von B miteinander unter Ausbildung eines Sechsrings ver- bunden zu einem Rest der Formel (a) oder (b) oder beide Wasserstoff oder ein Rest -COOM;
A ein Rest der Formel 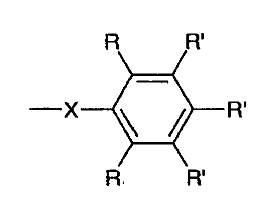
wobei die Reste A für n > 1 gleich oder verschieden sein können;
Y ein (Thio)Phenoxyrest der Formel 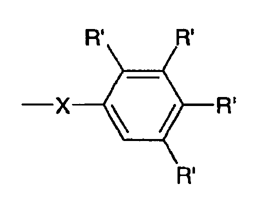 wobei die Reste Y für y > 1 gleich oder verschieden sein können;
X -O- oder -S-;
R gleiche oder verschiedene Reste: (i) C₁-C₃₀-Alkyl, wobei höchstens einer der Reste in der 1-Position ein ter- tiäres Kohlenstoffatom aufweisen darf, dessen Kohlenstoffkette durch ei- ne oder mehrere Gruppierungen -O-, -S-, -NR²- und/oder -CO- unterbrochen sein kann und das ein- oder mehrfach durch C₁-C₁₂-Alkoxy, Hydroxy und/oder Halogen substituiert sein kann; (ii) C₃-C₈-Cycloalkyl, wobei höchstens einer der Reste in der 1-Position ein tertiäres Kohlenstoffatom aufweisen darf, und das ein- oder mehrfach durch C₁-C₁₂-Alkyl und/oder C₁-C₁₂-Alkoxy substituiert sein kann; (iii) Aryl oder Hetaryl, das ein- oder mehrfach durch C₁-C₁₈-Alkyl und/oder C₁-C₁₂-Alkoxy substituiert sein kann; (iv) ein Rest -U-Aryl, der ein- oder mehrfach durch die vorstehenden, als Substituenten für die Arylreste (iii) genannten Reste substituiert sein kann, wobei U eine Gruppierung -O-, -S- oder -NR²- bedeutet; (v) C₁-C₁₂-Alkoxy, Hydroxy, Halogen oder Cyano
R' gleiche oder verschiedene Reste: Wasserstoff; einer der für R genannten Reste (i), (ii), (iii), (iv) und (v), wobei die Alkyl- reste (i) und die Cycloalkylreste (ii) in der 1-Position ein tertiäres Kohlen- stoffatom aufweisen können;
P ein über ein Stickstoffatom gebundener, 5- bis 9-gliedriger Ring, dessen Kohlenstoffkette durch eine oder mehrere Gruppierungen -O-, -S-, -NR²-, - CO- und/oder -SO₂- unterbrochen sein kann, an den ein oder zwei ungesät- tigte oder gesättigte 4- bis 8-gliedrige Ringe anneliert sein können, deren Kohlenstoffkette ebenfalls durch diese Gruppierungen und/oder -N= unter- brochen sein kann, wobei das gesamte Ringsystem ein- oder mehrfach substituiert sein kann durch: C₁-C₃₀-Alkyl, das durch Aryl, das durch C₁-C₁₈-Alkyl substituiert sein kann, ein- oder mehrfach substituiert sein kann; wobei die Reste P für p > 1 gleich oder verschieden sein können;
Hal Fluor, Chlor, Brom oder Iod;
M Wasserstoff, Alkalimetallkation, NH₄⁺ oder NR³₄⁺;
R¹ Wasserstoff C₁-C₃₀-Alkyl, das ein- oder mehrfach substituiert sein kann durch C₁-C₆- Alkoxy, Cyano und/oder Aryl; Phenyl, das ein- oder mehrfach substituiert sein kann durch C₁-C₁₈-Alkyl, C₁-C₆-Alkoxy, Halogen, Cyano und/oder Nitro;
R² Wasserstoff oder C₁-C₁₈-Alkyl;
R³ unabhängig voneinander Wasserstoff; C₁-C₁₈-Alkyl, das ein- oder mehrfach durch C₁-C₆-Alkoxy, Hydroxy, Halo- gen und/oder Cyano substituiert sein kann; Aryl oder Hetaryl, das jeweils ein- oder mehrfach durch C₁-C₆-Alkyl und/oder die vorstehenden, als Substituenten für Alkyl genannten Reste substituiert sein kann;
wobei für m = 0 (Perylenderivate)
n 1 bis 4
p 0 bis 2, wobei n + p ≤ 4;
y 0 bis 4, wobei n + p + y ≤ 4;
z 0 bis 4, wobei n + p + y + z ≤ 6;
und für m = 1 (Terrylenderivate)
n 1 bis 6;
p 0 bis 3; wobei n + p ≤ 6;
y 0 bis 3, wobei n + p + y ≤6;
z 0 bis 5, wobei n + p + y + z ≤ 8;
und für m = 2 (Quaterrylenderivate)
n 1 bis 8;
p 0 bis 5, wobei n + p ≤ 8;
y 0 bis 5, wobei n + p + y ≤ 8;
z 0 bis 6, wobei n + p + y ≤ 10.

2. Rylenderivate der allgemeinen Formel Ia nach Anspruch 1 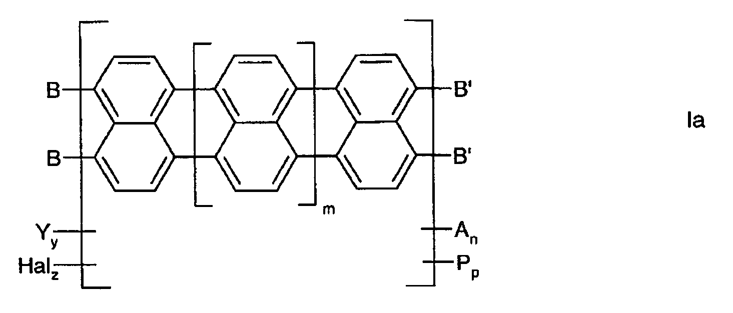 in der die Variablen folgende Bedeutung haben:
B miteinander unter Ausbildung eines Sechsrings verbunden zu einem Rest der Formel (a) oder (b) 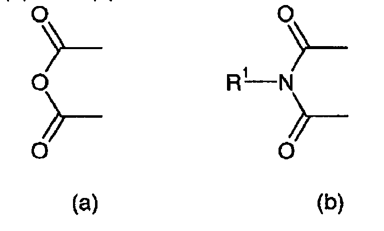
beide Wasserstoff oder ein Rest -COOM oder einer der beiden Reste ein Rest A oder Hal und der andere Rest Wasserstoff;
B' unabhängig von B miteinander unter Ausbildung eines Sechsrings ver- bunden zu einem Rest der Formel (a) oder (b) oder beide Wasserstoff oder ein Rest -COOM;
A ein Rest der Formel 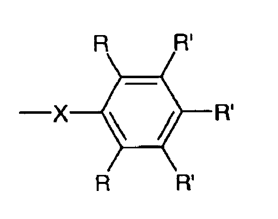 wobei die Reste A für n > 1 gleich oder verschieden sein können;
Y ein (Thio)Phenoxyrest der Formel 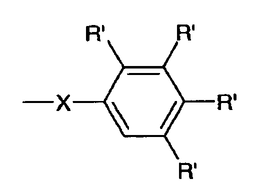 wobei die Reste Y für y > 1 gleich oder verschieden sein können;
X -O- oder -S-;
R gleiche oder verschiedene Reste:
(i) C₁-C₃₀-Alkyl, das in der 1-Position kein tertiäres Kohlenstoffatom auf- weist, dessen Kohlenstoffkette durch eine oder mehrere Gruppierungen -O-, -S-, -NR²- und/oder -CO- unterbrochen sein kann und das ein- oder mehrfach durch C1-C12-Alkoxy, Hydroxy und/oder Halogen substituiert sein kann;
(ii) C₃-C₈-Cycloalkyl, das in der 1-Position kein tertiäres Kohlenstoffatom aufweist und das ein- oder mehrfach durch C₁-C₁₈-Alkyl und/oder C₁-C₁₂- Alkoxy substituiert sein kann;
(iii) Aryl oder Hetaryl, das ein- oder mehrfach durch C₁-C₁₈-Alkyl und/oder C₁-C₁₂-Alkoxy substituiert sein kann;
(iv) ein Rest -U-Aryl, der ein- oder mehrfach durch die vorstehenden, als Substituenten für die Arylreste (iii) genannten Reste substituiert sein kann, wobei U eine Gruppierung -O-, -S- oder -NR². bedeutet;
(v) C₁-C₁₂-Alkoxy, Hydroxy, Halogen oder Cyano;
R' gleiche oder verschiedene Reste: Wasserstoff; einer der für R genannten Reste (i), (ii), (iii), (iv) und (v), wobei die Alkyl- reste (i) und die Cycloalkylreste (ii) in der 1-Position ein tertiäres Kohlen- stoffatom aufweisen können;
P ein über ein Stickstoffatom gebundener, 5- bis 9-gliedriger Ring, dessen Kohlenstoffkette durch eine oder mehrere Gruppierungen -O-, -S-, -NR²-, -CO- und/oder -SO₂- unterbrochen sein kann, an den ein oder zwei unge- sättigte oder gesättigte 4- bis 8-gliedrige Ringe anneliert sein können, de- ren Kohlenstoffkette ebenfalls durch diese Gruppierungen und/oder-N= unterbrochen sein kann, wobei das gesamte Ringsystem ein- oder mehr- fach substituiert sein kann durch: C₁-C₃₀-Alkyl, das durch Aryl, das durch C₁-C₁₈-Alkyl substituiert sein kann, ein- oder mehrfach substituiert sein kann; wobei die Reste P für p > 1 gleich oder verschieden sein können;
Hal Fluor, Chlor, Brom oder Iod;
M Wasserstoff, Alkalimetallkation, NH₄⁺ oder NR³₄⁺;
R₁ Wasserstoff; C₁-C₃₀-Alkyl, das ein- oder mehrfach substituiert sein kann durch C₁-C₆- Alkoxy, Cyano- und/oder Aryl; Phenyl, das ein- oder mehrfach substituiert sein kann durch C₁-C₁₈-Alkyl, C₁-C₆-Alkoxy, Halogen, Cyano und/oder Nitro;
R² Wasserstoff oder C₁-C₁₈-Alkyl;
R³ unabhängig voneinander Wasserstoff; C₁-C₁₈-Alkyl, das ein- oder mehrfach durch C₁-C₆-Alkoxy, Hydroxy, Halo- gen und/oder Cyano substituiert sein kann; Aryl oder Hetaryl, das jeweils ein- oder mehrfach durch C₁-C₆-Alkyl und/oder die vorstehenden, als Substituenten für Alkyl genannten Reste substituiert sein kann;
wobei für m = 0 (Perylenderivate)
n 1 bis 4
p 0 bis 2, wobei n + p ≤ 4;
y 0 bis 4, wobei n + p + y ≤ 4;
z 0 bis 4, wobei n + p + y + z ≤ 6;
und für m = 1 (Terrylenderivate)
n 1 bis 6;
p 0 bis 3; wobei n + p ≤ 6;
y 0 bis 3, wobei n + p + y ≤6;
z 0 bis 5, wobei n + p + y + z ≤ 8;
und für m = 2 (Quaterrylenderivate)
n 1 bis 8;
p 0 bis 5, wobei n + p ≤ 8;
y 0 bis 5. wobei n + p + y ≤ 8;
z 0 bis 6, wobei n + p + y ≤ 10.

3. Verwendung von Rylenderivaten gemäß Anspruch 1 oder 2 zur Einfärbung von organischen und anorganischen Materialien.

4. Verwendung nach Anspruch 3, **dadurch gekennzeichnet, daß** die organischen Materialien Lacke, Druckfarben oder Kunststoffe sind.

5. Verwendung von Rylenderivaten gemäß Anspruch 1 oder 2 zur Herstellung elektromagnetische Strahlung absorbierender und/oder emittierender wäßriger Polymerisatdispersionen.

6. Verwendung von Rylenderivaten gemäß Anspruch 1 oder 2 zur Erzeugung für das menschliche Auge nicht sichtbarer, Infrarotlicht absorbierender Markierungen und Beschriftungen.

7. Verwendung von Rylenderivaten gemäß Anspruch 1 oder 2 als Infrarotabsorber für das Wärmemanagement.

8. Verwendung von Rylenderivaten gemäß Anspruch 1 oder 2 als IR-Laserstrahlenabsorbierende Materialien beim Schweißbehandeln von Kunststoffteilen.

9. Verwendung von Rylenderivaten gemäß Anspruch 1 oder 2 zur Lasermarkierung und Laserbeschriftung.

10. Verwendung von Rylenderivaten gemäß Anspruch 1 oder 2 als Halbleiter in der organischen Elektronik.

11. Verwendung von Rylenderivaten gemäß Anspruch 1 oder 2 als Filter oder Emitter in Display-Anwendungen.

12. Verwendung von Rylenderivaten gemäß Anspruch 1 oder 2 als Emitter in Chemilumineszenzanwendungen.

13. Verwendung von Rylenderivaten gemäß Anspruch 1 oder 2 als Markierungsgruppen in Nachweisverfahren.

14. Verwendung von Rylenderivaten gemäß Anspruch 1 oder 2 als Aktivkomponenten in der Photovoltaik.

## Claims

1. A rylene derivative of the general formula Ia 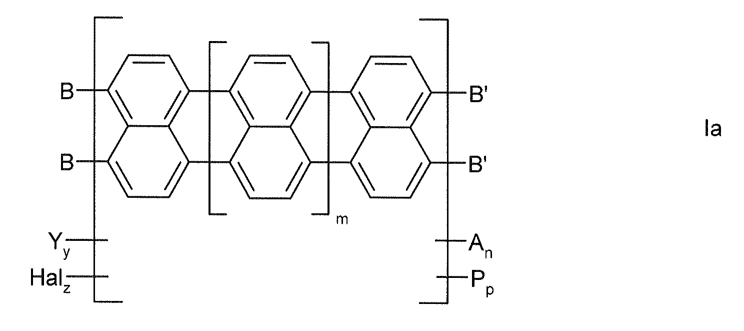 in which the variables are each defined as follows:
B are joined together with formation of a six- membered ring to give a radical of the formula (a) or (b) 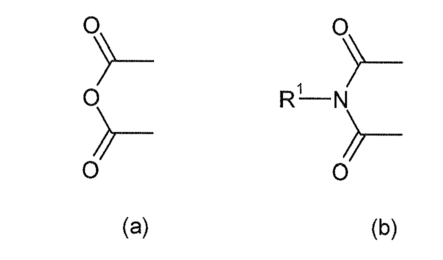 are both hydrogen or a -COOM radical, or one of the two radicals is an A radical or Hal and the other radical is hydrogen;
B', independently of B, are joined together with formation of a six-membered ring to give a radical of the formula (a) or (b), or are both hydrogen or a -COOM radical;
A is a radical of the formula 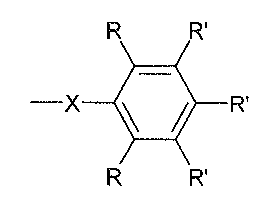 where the A radicals may be the same or different when n > 1;
Y is a (thio)phenoxy radical of the formula 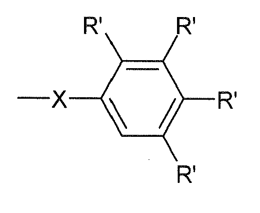 where the Y radicals may be the same or different when y > 1;
X is -O- or -S-;
R are identical or different radicals:
(i) C₁-C₃₀-alkyl, where not more than one of the radicals may have a tertiary carbon atom in the 1-position, and whose carbon chain may be interrupted by one or more -O-, -S-, -NR²- and/or -CO- moieties and which may be mono- or polysubstituted by C₁-C₁₂-alkoxy, hydroxy and/or halogen;
(ii) C₃-C₈-cycloalkyl, where not more than one of the radicals may have a tertiary carbon atom in the 1-position, and which may be mono- or polysubstituted by C₁-C₁₈-alkyl and/or C₁-C₁₂- alkoxy;
(iii) aryl or hetaryl which may be mono- or polysubstituted by C₁-C₁₈-alkyl and/or C₁-C₁₂- alkoxy;
(iv) a -U-aryl radical which may be mono- or polysubstituted by the above radicals specified as substituents for the aryl radicals (iii), where U is an -O-, -S- or -NR²- moiety;
(v) C₁-C₁₂-alkoxy, hydroxy, halogen or cyano
R' are identical or different radicals: hydrogen; one of the radicals specified for R (i), (ii), (iii), (iv) and (v), where the alkyl radicals (i) and the cycloalkyl radicals (ii) may have a tertiary carbon atom in the 1-position;
P is a 5- to 9-membered ring which is bonded via a nitrogen atom and whose carbon chain may be interrupted by one or more -0-, -S-, -NR²-, -CO- and/or -SO₂- moieties, to each of which may be fused one or two unsaturated or saturated 4- to 8- membered rings whose carbon chain may likewise be interrupted by these moieties and/or -N=, where the entire ring system may be mono- or polysubstituted by: C₁-C₃₀-alkyl which may be mono- or polysubstituted by aryl which may be substituted by C₁-C₁₈-alkyl; where the P radicals may be the same or different when p > 1;
Hal is fluorine, chlorine, bromine or iodine,
M is hydrogen, alkali metal cation, NH₄⁺ or NR³₄⁺;
R¹ is hydrogen; C₁-C₃₀-alkyl which may be mono- or polysubstituted by C₁-C₆-alkoxy, cyano and/or aryl; phenyl which may be mono- or polysubstituted by C₁-C₁₈-alkyl, C₁-C₆-alkoxy, halogen, cyano and/or nitro;
R² is hydrogen or C₁-C₁₈-alkyl;
R³ is independently hydrogen; C₁-C₁₈-alkyl which may be mono- or polysubstituted by C₁-C₆-alkoxy, hydroxy, halogen and/or cyano; aryl or hetaryl, each of which may be mono- or polysubstituted by C₁-C₆-alkyl and/or the above radicals specified as substituents for alkyl;
where, when m = 0 (perylene derivatives),
n is from 1 to 4
p is from 0 to 2 where n + p ≤ 4;
y is from 0 to 4 where n + p + y ≤ 4;
z is from 0 to 4 where n + p + y + z ≤ 6;
and, when m = 1 (terrylene derivatives),
n is from 1 to 6;
p is from 0 to 3 where n + p ≤ 6;
y is from 0 to 3 where n + p + y ≤6;
z is from 0 to 5 where n + p + y + z ≤ 8;
and, when m = 2 (quaterrylene derivatives),
n is from 1 to 8;
p is from 0 to 5 where n + p ≤ 8;
y is from 0 to 5 where n + p + p ≤ 8;
z is from 0 to 6 where n + p + y + z ≤ 10.

2. The rylene derivative of the general formula Ia according to claim 1 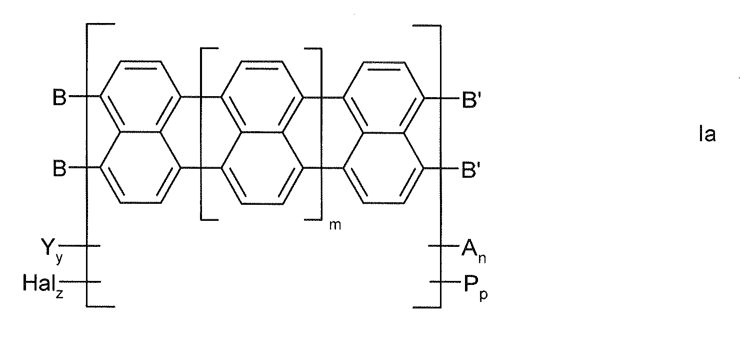 in which the variables are each defined as follows:
B are joined together with formation of a six- membered ring to give a radical of the formula (a) or (b) 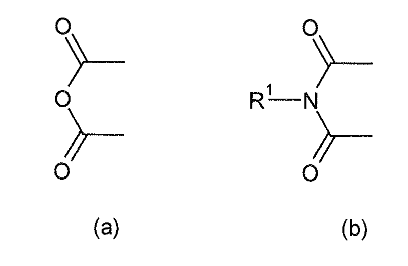 are both hydrogen or a -COOM radical, or one of the two radicals is an A radical or Hal and the other radical is hydrogen;
B', independently of B, are joined together with formation of a six-membered ring to give a radical of the formula (a) or (b), or are both hydrogen or a -COOM radical;
A is a radical of the formula 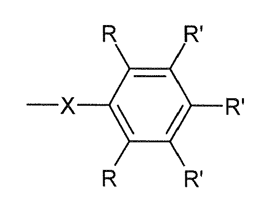 where the A radicals may be the same or different when n > 1;
Y is a (thio)phenoxy radical of the formula 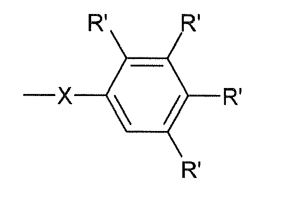 where the Y radicals may be the same or different when y > 1;
X is -0- or -S-;
R are identical or different radicals: (i) C₁-C₃₀-alkyl which does not have a tertiary carbon atom in the 1-position and whose carbon chain may be interrupted by one or more -O-, -S-, -NR²- and/or -CO- moieties and which may be mono- or polysubstituted by C₁-C₁₂-alkoxy, hydroxy and/or halogen; (ii) C₃-C₈-cycloalkyl which does not have a tertiary carbon atom in the 1-position and which may be mono- or polysubstituted by C₁-C₁₈-alkyl and/or C₁-C₁₂-alkoxy; (iii) aryl or hetaryl which may be mono- or polysubstituted by C₁-C₁₈-alkyl and/or C₁-C₁₂- alkoxy; (iv) a -U-aryl radical which may be mono- or polysubstituted by the above radicals specified as substituents for the aryl radicals (iii), where U is an -O-, -S- or -NR²- moiety; (v) C₁-C₁₂-alkoxy, hydroxy, halogen or cyano;
R' are identical or different radicals: hydrogen; one of the radicals specified for R (i), (ii), (iii), (iv) and (v), where the alkyl radicals (i) and the cycloalkyl radicals (ii) may have a tertiary carbon atom in the 1-position;
P is a 5- to 9-membered ring which is bonded via a nitrogen atom and whose carbon chain may be interrupted by one or more -0-, -S-, -NR²-, -CO- and/or -SO₂- moieties, to each of which may be fused one or two unsaturated or saturated 4- to 8- membered rings whose carbon chain may likewise be interrupted by these moieties and/or -N=, where the entire ring system may be mono- or polysubstituted by: C₁-C₃₀-alkyl which may be mono- or polysubstituted by aryl which may be substituted by C₁-C₁₈-alkyl; where the P radicals may be the same or different when p > 1;
Hal is fluorine, chlorine, bromine or iodine,
M is hydrogen, alkali metal cation, NH₄⁺ or NR³₄⁺;
R¹ is hydrogen; C₁-C₃₀-alkyl which may be mono- or polysubstituted by C₁-C₆-alkoxy, cyano and/or aryl; phenyl which may be mono- or polysubstituted by C₁-C₁₈-alkyl, C₁-C₆-alkoxy, halogen, cyano and/or nitro;
R² is hydrogen or C₁-C₁₈-alkyl;
R³ is independently hydrogen; C₁-C₁₈-alkyl which may be mono- or polysubstituted by C₁-C₆-alkoxy, hydroxy, halogen and/or cyano; aryl or hetaryl, each of which may be mono- or polysubstituted by C₁-C₆-alkyl and/or the above radicals specified as substituents for alkyl;
where, when m = 0 (perylene derivatives),
n is from 1 to 4
p is from 0 to 2 where n + p ≤ 4;
y is from 0 to 4 where n + p + y ≤ 4;
z is from 0 to 4 where n + p + y + z ≤ 6;
and, when m = 1 (terrylene derivatives),
n is from 1 to 6;
p is from 0 to 3 where n + p ≤ 6;
y is from 0 to 3 where n + p + y ≤ 6;
z is from 0 to 5 where n + p + y + z ≤ 8;
and, when m = 2 (quaterrylene derivatives),
n is from 1 to 8;
p is from 0 to 5 where n + p ≤ 8;
y is from 0 to 5 where n + p + y ≤ 8;
z is from 0 to 6 where n + p + y + z ≤ 10.

3. The use of rylene derivatives according to claim 1 or 2 for coloring organic and inorganic materials.

4. The use according to claim 3, wherein the organic materials are coatings, printing inks or plastics.

5. The use of rylene derivatives according to claim 1 or 2 for producing aqueous polymer dispersions which absorb and/or emit electromagnetic radiation.

6. The use of rylene derivatives according to claim 1 or 2 for obtaining markings and inscriptions which absorb infrared light and are invisible to the human eye.

7. The use of rylene derivatives according to claim 1 or 2 as infrared absorbers for heat management.

8. The use of rylene derivatives according to claim 1 or 2 as IR laser beam-absorbent materials in the fusion treatment of plastics parts.

9. The use of rylene derivatives according to claim 1 or 2 for laser marking and laser inscription.

10. The use of rylene derivatives according to claim 1 or 2 as semiconductors in organic electronics.

11. The use of rylene derivatives according to claim 1 or 2 as filters or emitters in display applications.

12. The use of rylene derivatives according to claim 1 or 2 as emitters in chemiluminescence applications.

13. The use of rylene derivatives according to claim 1 or 2 as labeling groups in detection methods.

14. The use of rylene derivatives according to claim 1 or 2 as active components in photovoltaics.

## Revendications

1. Dérivés Rylène de formule générale la 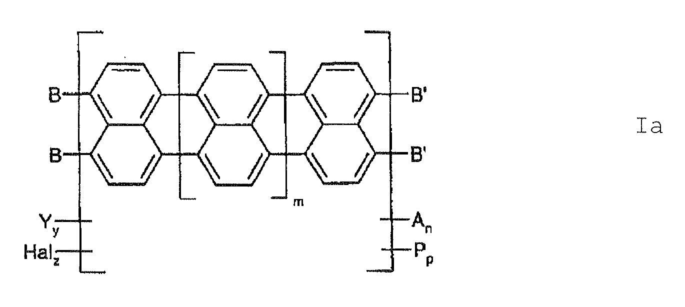 dans laquelle les variables ont les significations suivantes :
B sont liés l'un à l'autre avec formation d'un cycle hexagonal, en un radical de formule (a) ou (b) 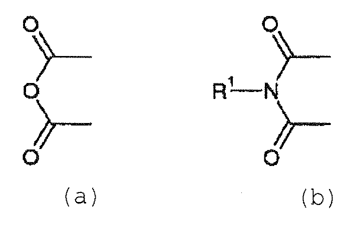 représentent l'un et l'autre un atome d'hydrogène ou un radical -COOM ou l'un des deux radicaux représente un radical A ou un atome d'halogène et l'autre radical représente un atome d'hydrogène ;
B' indépendamment de B sont liés l'un à l'autre avec formation d'un cycle hexagonal, en un radical de formule (a) ou (b), ou représentent l'un et l'autre un atome d'hydrogène ou un radical -COOM ;
A représente un radical de formule 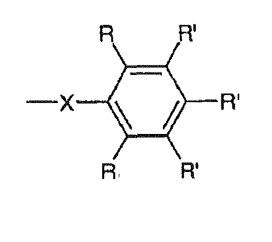 pour n > 1 les radicaux A pouvant être identiques ou différents ;
Y représente un radical (thio)phénoxy de formule 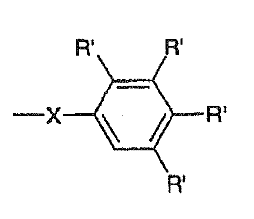 pour y > 1 les radicaux Y pouvant être identiques ou différents ;
X représente -O- ou -S- ;
R représente des radicaux identiques ou différents :
(i) un radical alkyle en C₁-C₃₀, au maximum un des radicaux pouvant comporter en la position 1 un atome de carbone tertiaire, dont la chaîne carbonée peut être interrompue par un ou plusieurs groupements -O-, -S-, -NR² et/ou -CO-, et qui peut être une ou plusieurs fois substitué par alcoxy en C₁-C₁₂, hydroxy et/ou halogène ;
(ii) un radical cycloalkyle en C₃-C₈, au maximum un des radicaux pouvant comporter en la position 1 un atome de carbone tertiaire, et qui peut être une ou plusieurs fois substitué par alkyle en C₁-C₁₈ et/ou alcoxy en C₁-C₁₂ ;
(iii) un radical aryle ou hétéroaryle, qui peut être une ou plusieurs fois substitué par alkyle en C₁-C₁₈ et/ou alcoxy en C₁-C₁₂ ;
(iv) un radical -U-aryle, qui peut être une ou plusieurs fois substitué par les radicaux précédents, nommés en tant que substituants pour les radicaux aryle (iii), U représentant un groupement -O-, -S- ou -NR²- ;
(v) un atome d'halogène ou un groupe alcoxy en C₁-C₁₂, hydroxy ou cyano ;
R' représente des radicaux identiques ou différents : un atome d'hydrogène ; l'un des radicaux (i), (ii), (iii), (iv) et (v) nommés pour R, les radicaux alkyle (i) et les radicaux cycloalkyle (ii) pouvant comporter en la position 1 un atome de carbone tertiaire ;
P représente un cycle à 5 à 9 chaînons, lié par un atome d'azote, dont la chaîne carbonée peut être interrompue par un ou plusieurs groupements -O-, -S-, -NR², -CO- et/ou -SO₂-, auquel peuvent être soudés un ou deux cycles saturés ou insaturés à 4 à 8 chaînons, dont la chaîne carbonée peut également être interrompue par ces groupements et/ou -N=, le système cyclique entier pouvant être une ou plusieurs fois substitué par : alkyle en C₁-C₃₀, qui peut être une ou plusieurs fois substitué par aryle, qui peut être substitué par alkyle en C₁-C₁₈ ; pour p > 1 les radicaux P pouvant être identiques ou différents ;
Hal représente le fluor, le chlore, le brome ou l'iode ;
M représente un atome d'hydrogène, un cation de métal alcalin, NH₄⁺ ou NR³₄⁺ ;
R¹ représente un atome d'hydrogène ; un radical alkyle en C₁-C₃₀, qui peut être une ou plusieurs fois substitué par alcoxy en C₁-C₆, cyano et/ou aryle ; un radical phényle, qui peut être une ou plusieurs fois substitué par alkyle en C₁-C₁₈, alcoxy en C₁-C₆, halogène, cyano et/ou nitro ;
R² représente un atome d'hydrogène ou un groupe alkyle en C₁-C₁₈ ;
R³ représente, chaque fois indépendamment, un atome d'hydrogène ; un radical alkyle en C₁-C₁₈, qui peut être une ou plusieurs fois substitué par alcoxy en C₁-C₆, hydroxy, halogène et/ou cyano ; un radical aryle ou hétéroaryle, chacun pouvant être une ou plusieurs fois substitué par alkyle en C₁-C₆ et/ou les radicaux nommés précédemment en tant que substituants pour alkyle ;
pour m = 0 (dérivés pérylène)
n vaut de 1 à 4 ;
p vaut de 0 à 2, n + p étant ≤ 4 ;
y vaut de 0 à 4, n + p + y étant ≤ 4 ;
z vaut de 0 à 4, n + p + y + z étant ≤ 6 ;
et pour m = 1 (dérivés terrylène)
n vaut de 1 à 6 ;
p vaut de 0 à 3, n + p étant ≤ 6 ;
y vaut de 0 à 3, n + p + y étant ≤ 6 ;
z vaut de 0 à 5, n + p + y + z étant ≤ 8 ;
et pour m = 2 (dérivés quaterrylène)
n vaut de 1 à 8 ;
p vaut de 0 à 5, n + p étant ≤ 8 ;
y vaut de 0 à 5, n + p + y étant ≤ 8 ;
z vaut de 0 à 6, n + p + y + z étant ≤ 10.

2. Dérivés Rylène de formule générale Ia selon la revendication 1 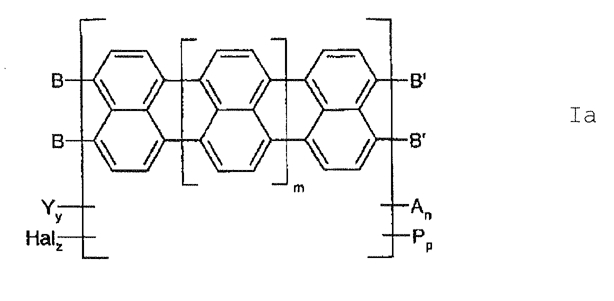 dans laquelle les variables ont les significations suivantes :
B sont liés l'un à l'autre avec formation d'un cycle hexagonal, en un radical de formule (a) ou (b) 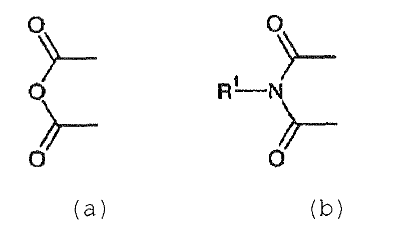 représentent l'un et l'autre un atome d'hydrogène ou un radical -COOM ou l'un des deux radicaux représente un radical A ou un atome d'halogène et l'autre radical représente un atome d'hydrogène ;
B' indépendamment de B sont liés l'un à l'autre avec formation d'un cycle hexagonal, en un radical de formule (a) ou (b), ou représentent l'un et l'autre un atome d'hydrogène ou un radical -COOM ;
A représente un radical de formule 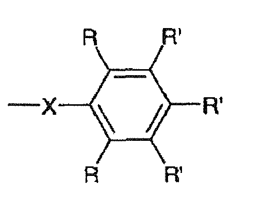 pour n > 1 les radicaux A pouvant être identiques ou différents ;
Y représente un radical (thio)phénoxy de formule 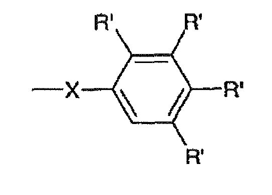 pour y > 1 les radicaux Y pouvant être identiques ou différents ;
X représente -O- ou -S- ;
R représente des radicaux identiques ou différents : (i) un radical alkyle en C₁-C₃₀, qui ne comporte en la position 1 aucun atome de carbone tertiaire, dont la chaîne carbonée peut être interrompue par un ou plusieurs groupements -O-, -S-, -NR² et/ou - CO-, et qui peut être une ou plusieurs fois substitué par alcoxy en C₁-C₁₂, hydroxy et/ou halogène ; (ii) un radical cycloalkyle en C₃-C₈, qui ne comporte en la position 1 aucun atome de carbone tertiaire, et qui peut être une ou plusieurs fois substitué par alkyle en C₁-C₁₈ et/ou alcoxy en C₁-C₁₂ ; (iii) un radical aryle ou hétéroaryle, qui peut être une ou plusieurs fois substitué par alkyle en C₁-C₁₈ et/ou alcoxy en C₁-C₁₂ ; (iv) un radical -U-aryle, qui peut être une ou plusieurs fois substitué par les radicaux précédents, nommés en tant que substituants pour les radicaux aryle (iii), U représentant un groupement -O-, -S- ou -NR²- ; (v) un atome d'halogène ou un groupe alcoxy en C₁-C₁₂, hydroxy ou cyano ;
R' représente des radicaux identiques ou différents : un atome d'hydrogène ; l'un des radicaux (i), (ii), (iii), (iv) et (v) nommés pour R, les radicaux alkyle (i) et les radicaux cycloalkyle (ii) pouvant comporter en la position 1 un atome de carbone tertiaire ;
P représente un cycle à 5 à 9 chaînons, lié par un atome d'azote, dont la chaîne carbonée peut être interrompue par un ou plusieurs groupements -O-, -S-, -NR², -CO- et/ou -SO₂-, auquel peuvent être soudés un ou deux cycles saturés ou insaturés à 4 à 8 chaînons, dont la chaîne carbonée peut également être interrompue par ces groupements et/ou -N=, le système cyclique entier pouvant être une ou plusieurs fois substitué par : alkyle en C₁-C₃₀, qui peut être une ou plusieurs fois substitué par aryle, qui peut être substitué par alkyle en C₁-C₁₈ ; pour p > 1 les radicaux P pouvant être identiques ou différents ;
Hal représente le fluor, le chlore, le brome ou l'iode ;
M représente un atome d'hydrogène, un cation de métal alcalin, NH₄⁺ ou NR³₄⁺;
R¹ représente un atome d'hydrogène ; un radical alkyle en C₁-C₃₀, qui peut être une ou plusieurs fois substitué par alcoxy en C₁-C₆, cyano et/ou aryle ; un radical phényle, qui peut être une ou plusieurs fois substitué par alkyle en C₁-C₁₈, alcoxy en C₁-C₆, halogène, cyano et/ou nitro ;
R² représente un atome d'hydrogène ou un groupe alkyle en C₁-C₁₈ ;
R³ représente, chaque fois indépendamment, un atome d'hydrogène ; un radical alkyle en C₁-C₁₈, qui peut être une ou plusieurs fois substitué par alcoxy en C₁-C₆, hydroxy, halogène et/ou cyano ; un radical aryle ou hétéroaryle, chacun pouvant être une ou plusieurs fois substitué par alkyle en C₁-C₆ et/ou les radicaux nommés précédemment en tant que substituants pour alkyle ;
pour m = 0 (dérivés pérylène)
n vaut de 1 à 4 ;
p vaut de 0 à 2, n + p étant ≤ 4 ;
y vaut de 0 à 4, n + p + y étant ≤ 4 ;
z vaut de 0 à 4, n + p + y + z étant ≤ 6 ;
et pour m = 1 (dérivés terrylène)
n vaut de 1 à 6 ;
p vaut de 0 à 3, n + p étant ≤ 6 ;
y vaut de 0 à 3, n + p + y étant ≤ 6 ;
z vaut de 0 à 5, n + p + y + z étant ≤ 8 ;
et pour m = 2 (dérivés quaterrylène)
n vaut de 1 à 8 ;
p vaut de 0 à 5, n + p étant ≤ 8 ;
y vaut de 0 à 5, n + p + y étant ≤ 8 ;
z vaut de 0 à 6, n + p + y + z étant ≤ 10.

3. Utilisation de dérivés Rylène selon la revendication 1 ou 2, pour la coloration de matières organiques et de matières inorganiques.

4. Utilisation selon la revendication 3, **caractérisée en ce que** les matières organiques sont des peintures, des encres d'impression ou des matières plastiques.

5. Utilisation de dérivés Rylène selon la revendication 1 ou 2, pour la préparation de dispersions aqueuses de polymérisats émettant et/ou absorbant un rayonnement électromagnétique.

6. Utilisation de dérivés Rylène selon la revendication 1 ou 2, pour la production de marquages ou d'inscriptions absorbant dans l'infrarouge, invisibles à l'oeil nu.

7. Utilisation de dérivés Rylène selon la revendication 1 ou 2, en tant qu'absorbeur infrarouge pour la gestion de la chaleur.

8. Utilisation de dérivés Rylène selon la revendication 1 ou 2, en tant que matériaux absorbant les rayons laser-IR dans le traitement par soudage de pièces en matière plastique.

9. Utilisation de dérivés Rylène selon la revendication 1 ou 2, pour le marquage au laser et l'inscription laser.

10. Utilisation de dérivés Rylène selon la revendication 1 ou 2, en tant que semi-conducteur en électronique organique.

11. Utilisation de dérivés Rylène selon la revendication 1 ou 2, sous forme de filtres ou d'émetteurs pour des applications d'affichage.

12. Utilisation de dérivés Rylène selon la revendication 1 ou 2, sous forme d'émetteurs dans des applications de chimiluminescence.

13. Utilisation de dérivés Rylène selon la revendication 1 ou 2, en tant que groupes de marquage dans des procédés de détection.

14. Utilisation de dérivés Rylène selon la revendication 1 ou 2, en tant que composants actifs dans la technique photovoltaïque.
